(19)

Europäisches
Patentamt

European
Patent Office

Office européen
des brevets

(11) **EP 4 623 891 A2**

(12) **EUROPEAN PATENT APPLICATION**

(43) Date of publication:
**01.10.2025 Bulletin 2025/40**

(21) Application number: **25189565.2**

(22) Date of filing: **21.08.2020**

(51) International Patent Classification (IPC):
***A61K 8/02*** (2006.01)

(52) Cooperative Patent Classification (CPC):
**A61Q 19/00; A61K 8/0204; A61K 8/44;
A61Q 19/08;** A61K 2800/5922

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.08.2019 KR 20190102243**

(62) Document number(s) of the earlier application(s) in
accordance with Art. 76 EPC:
**20854144.1 / 4 018 996**

(71) Applicant: **LG Household & Health Care Ltd.
Seoul 03184 (KR)**

(72) Inventors:
• **KWON, Koo Chul**
**07795 Seoul (KR)**
• **KWON, Tae Geun**
**07795 Seoul (KR)**

• **PARK, Sang Wook**
**07795 Seoul (KR)**
• **LEE, So Young**
**07795 Seoul (KR)**
• **SEO, Ji Hyun**
**07795 Seoul (KR)**

(74) Representative: **Goddar, Heinz J.
Boehmert & Boehmert
Anwaltspartnerschaft mbB
Pettenkoferstrasse 22
80336 München (DE)**

Remarks:
This application was filed on 15.07.2025 as a
divisional application to the application mentioned
under INID code 62.

(54) **COSMETIC COMPOSITION COMPRISING EUTECTIC MIXTURE**

(57)    The present invention relates to a cosmetic composition comprising a eutectic mixture containing: an amino acid, an alpha-hydroxy acid, and water; or two types of amino acids. The cosmetic comprises either an amino acid and an alpha-hydroxy acid or two types of amino acids in the form of a eutectic mixture. Thus, a cosmetic composition with excellent skin penetration effect even under weakly acidic or neutral pH conditions, which causes less irritation, can be provided, wherein the cosmetic composition provides excellent keratin exfoliation, reduces the number of pores, enhances skin elasticity, and promotes skin regeneration while having excellent low-temperature stability.

【FIG.1】

EP 4 623 891 A2

**Description**

[Technical Field]

The present invention a cosmetic composition including a eutectic mixture containing an alpha-hydroxy acid, an amino acid, and water; and a cosmetic composition including a eutectic mixture containing a first amino acid and a second amino acid, and more specifically, to a cosmetic composition which has high skin penetrability and an excellent keratin exfoliation effect and low-temperature stability even under pH conditions that cause less irritation on the skin and maintains the keratin exfoliation effect even under severe conditions by including a eutectic mixture of water, an amino acid and an alpha-hydroxy acid, or a eutectic mixture of a first amino acid and a second amino acid.

[Background Art]

An alpha-hydroxy acid ($\alpha$-hydroxy acid, hereinafter, referred to as AHA) is one of the ingredients commonly used for softening or removing the hyperkeratinized skin layer, and exhibits skin-improving effects such as promotion of keratin turnover, collagen synthesis, skin moisturization enhancement, fine wrinkle relief, and acne treatment and prevention (Journal of the Korean Society for Wellness, Vol. 10, No. 2, 2015, 161-169). Although most AHAs are excellent in skin permeability and a skin improvement effect under low pH conditions, it is difficult to use a high content of the AHA because skin irritation such as itching, tingling and hot flashes due to low pH and various inflammatory irritations such as erythema and edema are severe. Further, the AHA has a disadvantage in that when AHA is dissolved in water, AHA is ionized which slows down the skin's absorption of AHA. In particular, when AHA is formulated as a cosmetic product, the pH of the formulation becomes low, so the stability becomes low and negative effects such as irritation to the skin may occur. When the pH of the formulation is increased using a neutralizing agent to compensate for this problem, there is a problem in that the effect of AHA is reduced.

In addition, amino acids that exhibit exfoliating action on dry skin together with the AHA are also widely used in skin applicable cosmetics (Tatsuya Ozawa et al., The role of humectants in skin moisture retention. Skin Research 27, 276-288 (1985); and KR Registered Patent No. 10-1508168). However, it is known through [S. E. coli Wolverton, $\alpha$-hydroxy acids, In Comprehensive Dermatologic Drug Therapy, 3rd edition, Elsevier, 570 (2012)] that the skin permeability of the AHA and amino acids is remarkably decreased at weakly acidic or neutral pH, and that the AHA and amino acids may cause discomfort at strongly acidic pH such as skin irritation to people with particularly sensitive skin.

Meanwhile, the eutectic phenomenon is a phenomenon in which the melting point drops while two or more materials are mixed, so even though each component contained in the mixture shows a solid state at room temperature the mixture may become an anhydrous solution rather than a solid crystal, which is due to Van der Waals interactions or hydrogen bonding between the components. Thus, when the AHA is selected as one of the components contained in the eutectic mixture, it is possible to obtain an anhydrous liquid. Since the AHA is not ionized in an anhydrous state, the characteristics of an acid are not exhibited, so the AHA can be used with less irritation, and also the non-charged AHA is advantageous for skin penetration.

However, such eutectic mixtures may not be stable in cosmetics. When a protic solvent capable of forming hydrogen bonds is added to a composition containing the eutectic mixture, it is difficult to maintain the characteristics of the eutectic mixture because the solvent interacts (hydrogen-bonds) with each component of the eutectic mixture thus interfering with the bond between the constituents of the eutectic mixture. Furthermore, although a anhydrous formulation is suitable for cosmetics containing a eutectic mixture, most eutectic mixtures have a higher specific gravity than water, and thus cause precipitation when mixed with oil, so it is difficult to evenly disperse the eutectic mixture on oils with a low viscosity. Prior solutions for this problem was to add a hydrophilic powder to the composition, but there was a problem in that the hydrophilic eutectic mixture adsorbed and aggregated on the surface of the hydrophilic powder, making it difficult to evenly disperse the hydrophilic eutectic mixture.

Therefore, under this background, the present inventors have studied and developed a cosmetic composition in which the irritation problem due to low pH is alleviated and formation stability is improved while a eutectic mixture contains water, the AHA and an amino acid; or two types of amino acids, thereby completing the present invention.

[Disclosure]

[Technical Problem]

The present inventors have studied to develop a cosmetic composition that solves a problem in that an AHA imparts irritation at low pH and a problem in that the AHA or an amino acid has low skin permeation when applied at skin pH, and as a result, confirmed that by adding water, the AHA and an amino acid; two types of amino acids; or water and two types of amino acids in the form of a eutectic mixture to a cosmetic composition, the skin permeability of the AHA or the amino acid could be improved without skin irritation at weakly acidic or neutral pH, the amino acid could be stably and uniformly maintained without being precipitated even at low temperature, and the keratin exfoliation effect could be maintained even under severe conditions (for example, high temperature, high pressure, and the like), thereby completing the present invention.

Therefore, an object of the present invention is to provide a cosmetic composition which has excellent keratin exfoliation effect, cause less irritation, has excellent low-temperature stability under weakly acidic or neutral conditions, and maintains excellent keratin exfoliation effect even under severe conditions by including water, the AHA and the amino acid; two types of amino acids; or water and two types of amino acids in the form of a eutectic mixture in the cosmetic composition.

[Technical Solution]

As a means for solving the above problems, the cosmetic composition of the present invention provides a cosmetic composition including a eutectic mixture containing: water, an amino acid, and an alpha-hydroxy acid (AHA); or a first amino acid and a second amino acid.

In one exemplary embodiment, the cosmetic composition of the present invention includes a eutectic mixture containing: water; an amino acid; and an $\alpha$-hydroxy acid (hereinafter, referred to as AHA).

In the present invention, the alpha-hydroxy acid (AHA) and the amino acid may each be contained in a eutectic mixture as a use for a skin improvement effect such as promotion of keratin exfoliation, alleviation of fine wrinkles, alleviation of acne, or improvement of facial skin tone.

In the present invention, the alpha-hydroxy acid is a general term for a compound in which an alcohol group or a hydroxyl group is added to the carbon at the $\alpha$ position of a carboxylic acid, and serves to remove hyperkeratinization by lowering the ionic bonding energy of keratinocytes or to improve the skin by promoting the formation of new cells to shorten the period of the keratinization process that is extended as the skin ages, thereby promoting the activity of skin cells. Further, AHA makes the skin elastic and soft by promoting the production of collagen and elastin by fibroblasts, and may enhance skin moisturization by promoting the production of a mucopolysaccharide which is an important extracellular matrix component of the dermis. The AHA helps to remove a linker between the keratins in the stratum corneum by the action of acid, and exhibits the effect of gently dissolving the keratin. The AHA may be one or more selected from the group consisting of lactic acid contained in spoiled milk or tomato juice and the like; citric acid contained in mandarin oranges, oranges and the like; malic acid contained in apples, and the like; tartaric acid contained in wines and the like; glycolic acid contained in sugar cane and the like; and mandelic acid, but is not limited thereto.

In one exemplary embodiment, the AHA may be lactic acid or malic acid. The lactic acid penetrates into the skin quickly due to a small molecular weight among AHAs, and thus can implement a desired effect of the AHA, while the malic acid has a plurality of carboxyl groups which may induce stronger bonds when forming a eutectic mixture, and has a molecular structure similar to that of an amino acid, particularly serine, which is advantageous in forming a structurally stable eutectic mixture in the form of a dimer or a multimer.

In an exemplary embodiment of the present invention, when AHA is contained in the eutectic mixture, the AHA may be included in an amount of 1 to 50 parts by weight, for example, 5 to 40 parts by weight, 10 to 30 parts by weight, 15 to 20 parts by weight, 3 to 25 parts by weight, 5 to 20 parts by weight, or 6 to 18 parts by weight with respect to 100 parts by weight of the eutectic mixture. When AHA is contained in the eutectic mixture in an amount of less than 1 part by weight, the desired effect cannot be exhibited, and when AHA is contained in the eutectic mixture in an amount of more than 50 parts by weight, skin irritation may occur.

When AHA is contained in the eutectic mixture as described above, the eutectic mixture may be prepared by mixing the AHA with an amino acid, which is a component that can be bound by a dipole-dipole interaction between polar molecules, a dipole-induced dipole interaction, a hydrogen bond or a Van der Waals interaction. In this case, the AHA may act as a hydrogen bond donor (HBD), and the amino acid may act as a hydrogen bond acceptor (HBA). For example, the amino acid may include alanine, cysteine, aspartic acid, glutamic acid, phenylalanine, glycine, histidine, isoleucine, lysine, leucine, methionine, asparagine, pyrrolysine, proline, glutamine, arginine, serine, threonine, selenocysteine, valine, tryptophan or tyrosine.

In the present invention, the total content of an amino acid in the eutectic mixture may be 1 to 75 parts by weight, 5 to 70 parts by weight, 10 to 60 parts by weight, 15 to 50 parts by weight, 20 to 47 parts by weight, 10 to 70 parts by weight, 20 to 65 parts by weight, 30 to 60 parts by weight, 35 to 55 parts by weight, 40 to 50 parts by weight, or 42 to 47 parts by weight based on the total 100 parts by weight of the eutectic mixture, but is not limited thereto. When the amino acid is contained in the eutectic mixture in an amount of less than 1 part by weight, the desired effect cannot be exhibited, and when the amino acid is contained in the eutectic mixture in an amount of more than 75 parts by weight, skin irritation and amino acid precipitation in the composition may occur. In one exemplary embodiment, the amino acid included in the eutectic mixture may be serine, proline, threonine, arginine or cysteine.

In the present invention, the eutectic mixture may be prepared from AHA and an amino acid, preferably from water, AHA and an amino acid.

In the present invention, the AHA and the amino acid may be included in the eutectic mixture at a weight ratio of 1:0.1 to 1: 10, for example, 1:0.3 to 1:5, 1:0.5 to 1:3, 1:0.7 to 1:2, 1:3 to 1:10, or 1:5 to 1:8.

Generally, in the presence of water, AHA in the eutectic mixture is ionized, and thus may increase skin irritation. Thus, conventional eutectic mixtures had to be subjected to a process of removing a solvent (water) by an additional process such as evaporation, cooling or lyophilization. However, since the eutectic mixture according to the present invention is not ionized even in the presence of water through intermolecular bonding between AHA and an amino acid such as a dipole-dipole interaction, a dipole-induced dipole interaction, a hydrogen bond or a Van der Waals interaction, the eutectic mixture may be safe from negative effects such as an increase in skin irritation.

Thus, the eutectic mixture according to the present invention includes water. The eutectic mixture of the present invention has a low melting point of 0°C or less by including water, and the amino acid component may be stable in the composition. Further, since the inclusion of water in the eutectic mixture exposes AHA and an amino acid to excessive water, the bonds may be gradually dissociated, thereby exhibiting a skin improvement effect by promoting exfoliation of the keratin. Therefore, the eutectic mixture of the present invention can maintain a liquid state uniformly and stably without precipitation of an amino acid even under various environmental changes.

In the present invention, the water may be included in an amount of 25 to 80 parts by weight with respect to the total 100 parts by weight of the eutectic mixture. When the content of water in the eutectic mixture is less than 25 parts by weight, the amino acid may not be stable because the melting point of the eutectic mixture cannot be reduced, and when the content is more than 80 parts by weight, it may be difficult to implement the desired effect because the skin permeability of AHA and the amino acid decreases and the content of the amino acid decreases due to the lowered hydrogen bond stability. For example, the eutectic mixture of the present invention may include 30 to 75 parts by weight, 35 to 70 parts by weight, 40 to 65 parts by weight, or 50 to 55 parts by weight of water based on the total weight of the eutectic mixture, but the parts by weight is not limited thereto.

In another exemplary embodiment, the cosmetic composition of the present invention includes a eutectic mixture containing a first amino acid and a second amino acid.

In the present invention, the first amino acid and the second amino acid may be one or more selected from the group consisting of alanine, cysteine, aspartic acid, glutamic acid, phenylalanine, glycine, histidine, isoleucine, lysine, leucine, methionine, asparagine, pyrrolysine, proline, glutamine, arginine, serine, threonine, selenocysteine, valine, tryptophan and tyrosine. For example, the first amino acid and the second amino acid may be two types selected from the group consisting of serine, arginine and glutamic acid.

In the exemplary embodiment, the total content of amino acids in the eutectic mixture may be 1 to 75 parts by weight, 5 to 70 parts by weight, 10 to 60 parts by weight, 15 to 50 parts by weight, 20 to 47 parts by weight, 10 to 70 parts by weight, 20 to 65 parts by weight, 30 to 60 parts by weight, 35 to 55 parts by weight, 40 to 50 parts by weight, or 42 to 47 parts by weight based on the total 100 parts by weight of the eutectic mixture, but is not limited thereto. When the amino acids are contained in the eutectic mixture in an amount of less than 1 part by weight, the desired effect cannot be exhibited, and when the amino acids are contained in the eutectic mixture in an amount of more than 75 parts by weight, skin irritation and amino acid precipitation in the composition may occur. In this case, the first amino acid may be included in an amount of 1 to 50 parts by weight based on the total 100 parts by weight of the eutectic mixture, and the second amino acid may be included in an amount excluding the parts by weight of the first amino acid from 75 parts by weight. For example, the second amino acid may be 1 to 25 parts by weight. In the present invention, the first amino acid may be 1 to 50 parts by weight, preferably 5 to 40 parts by weight, 10 to 35 parts by weight, or 15 to 30 parts by weight.

In addition, the eutectic mixture of the present invention may further include water. Water may be included in an amount of 25 to 80 parts by weight based on the total 100 parts by weight of the eutectic mixture. The above-described content may be applied as it is to the water contained in the eutectic mixture.

In the present invention, the eutectic mixture may be prepared from a first amino acid and a second amino acid, preferably from water, a first amino acid and a second amino acid.

As described above, the eutectic mixture including the first amino acid and the second amino acid may include 25 to 80 parts by weight of water, 1 to 50 parts by weight of the first amino acid, and 1 to 25 parts by weight of the second amino acid based on the total 100 parts by weight of the eutectic mixture.

In the following examples, it was confirmed that the eutectic mixture including two types of amino acids exhibited skin improvement effects such as reducing the number of pores, alleviating wrinkles, promoting skin regeneration, increasing the total amount of collagen and enhancing skin elasticity, and it could be confirmed that the skin improvement effects as described above were maintained even after storage under severe conditions. Furthermore, it was confirmed that the eutectic mixture containing only two types of amino acids and water without AHA showed excellent skin permeability, keratin exfoliation effect, low temperature stability, and the like even under weakly acidic or neutral acidity conditions.

As used herein, "eutectic mixture" refers to a mixture of two or more solid or liquid materials, and two components having a high melting point are mixed to form a composite by a dipole-dipole interaction between polar molecules, a dipole-induced dipole interaction, and an intermolecular hydrogen bond or Van der Waals interaction. The composite formed as described above can show a liquid state at room temperature by hindering the ability of each component to crystallize, that is, the ability of each component to return to a solid state, to have a melting point lower than the component having the lowest melting point. In the present invention, since the eutectic mixture includes water, the melting point becomes lower than 0 °C and a stable composition may be maintained even in various environments.

Further, in the eutectic mixture, polar compounds may be bonded to each other to form a eutectic mixture. The polar compound may be a polar molecule or a charged molecule. In addition, there must be a difference between the polarities among the polar compounds, and the same compound cannot form a eutectic mixture. More specifically, the pKa or pI value between the components constituting the eutectic mixture may have a difference of, for example, 1.0 or more, 2.0 or more, 3.0 or more, 4.0 or more, 5.0 or more, 6.0 or more, or 7.0 or more. In the case where the component constituting the eutectic mixture is a component having a plurality of pKa values, it may be selected based on the pKa1 value.

More specifically, the polar compound may be selected from AHA or an amino acid (AA). Even more specifically, when one of the components constituting the eutectic mixture is AHA, as the other compound, an amino acid having a pI higher than the pKa1 value of the AHA may be selected. For example, the difference between the pKa1 value and the pI value between the AHA and the amino acid constituting the eutectic mixture may be 1.0 or more, 2.0 or more, 3.0 or more, 4.0 or more, 5.0 or more, 6.0 or more, or 7.0 or more. Even more specifically, when one of the components constituting the eutectic mixture is an amino acid, as the other compound, an amino acid having a pI higher or lower than the pI value of the amino acid may be selected. For example, the difference between the pI values of the two amino acids constituting the eutectic mixture may be 1.0 or more, 2.0 or more, 3.0 or more, 4.0 or more, 5.0 or more, 6.0 or more, or 7.0 or more.

More specifically, the polar compound may be selected from a polar amino acid (polar AA), a positively charged amino acid (+ charged AA) and a negatively charged amino acid (- charged AA). For example, it is possible to form a eutectic mixture with any one of the polar amino acids and any one of the positively charged amino acids. For example, it is possible to form a eutectic mixture with any one of the polar amino acids and any one of the negatively charged amino acids. For example, it is possible to form a eutectic mixture with any one of the positively charged amino acids and any one of the negatively charged amino acids. The polar amino acid refers to any one of threonine, tyrosine, glutamine, serine and asparagine. The positively charged amino acid refers to any one of histidine, lysine and arginine. The negatively charged amino acid refers to any one of aspartic acid and glutamic acid.

In the eutectic mixture, a ratio between materials required for the eutectic mixture may be determined according to the charge of the molecules of the components constituting the eutectic mixture, the type and number of functional groups of the molecule, and the polarity of the molecule or functional group. Furthermore, the ratio between materials required for the eutectic mixture may be determined according to the size or similarity of the molecules. For example, arginine and glutamic acid, arginine and aspartic acid, lysine and glutamic acid, lysine and aspartic acid, arginine and asparagine, and arginine and glutamine may form a eutectic mixture at a molar ratio of 1:1. For example, arginine and serine, arginine and threonine, arginine and tyrosine, arginine and asparagine, and arginine and glutamine may form a eutectic mixture at a molar ratio of 1:2.

In the present invention, the eutectic mixture may be used interchangeably as a term having the same meaning as "eutectic solvent", "eutectic body" or "eutectic mixture solution".

In general, in the case of a eutectic mixture, the effect exhibited by the eutectic mixture is reduced when the eutectic mixture is broken under severe conditions such as high temperature. When the keratin exfoliation effects of the cosmetic composition including the eutectic mixture according to the present invention (A) and the cosmetic composition which is A but has been kept in a high temperature for a predetermined period of time (B) are compared, the effect of A may be 5%, preferably 10%, more preferably 12%, and most preferably 15% or more than that of B. Further, when the skin permeabilities of the cosmetic composition including the eutectic mixture according to the present invention (A) and the cosmetic composition which is A but has been kept in a high temperature for a predetermined period of time (B) are compared, the skin permeability of A may be 5%, preferably 10%, more preferably 12%, and most preferably 15% or more than that of B. In this case, the cosmetic composition kept in a high temperature for a predetermined period of time refers to a cosmetic composition stored at 50°C for 6 weeks or at 60°C for 5 weeks or more.

The acidity of the eutectic mixture according to the present invention may be adjusted to have a value of pH 3.5 to pH 10 in order to alleviate discomfort such as skin irritation when applied to the skin. The eutectic mixture according to the present invention can be structurally prepared in a section in which both an amine group of the amino acid or a carboxyl group of the AHA are charged, and thus the section may have a pH of 3.5 or more. For example, the eutectic mixture may be adjusted to have weak acidity to neutral acidity, such as pH 4 to pH 9, pH 5 to pH 8, and pH 6 to pH 7.

The eutectic mixture of the present invention may include water, AHA and an amino acid; or two types of amino acids to lower the solubility at low temperature due to the melting point lowering properties of the eutectic mixture, thereby enhancing low-temperature stability. In addition, since the skin permeability of AHA and/or the amino acid is rapidly decreased as the pH is generally increased, conventional cosmetics have maintained a low pH. However, the eutectic mixture of the present invention can maintain high skin permeability of AHA and/or the amino acid as at low pH while reducing skin irritation that may occur at weak acidity to neutral acidity.

The eutectic mixture according to the present invention may be prepared by selecting any method commonly used by those skilled in the art, and then adding water to the method, and an exemplary method of preparing the eutectic mixture is as follows:

i) a method in which the component having the lowest melting point among the components constituting the eutectic mixture is heated to the melting temperature of the component to make it liquid, and then the remaining components are completely dissolved in the molten liquid, and

ii) a method in which the the components constituting the eutectic mixture are dissolved in a solvent, where in the solvent is one which can completely dissolve at least one component, and dissolving it at a high temperature of 50°C or higher until it becomes homogeneous.

In this case, for the method of ii), the solvent may be evaporated according to the need for the solvent.

In one exemplary embodiment, the eutectic mixture of the present invention may be prepared by the method of ii).

As a specific example of ii), a sample is prepared in a reaction vessel that require heating and a homo-disper. Without being limited by the material of the reaction vessel, for example, a glass beaker or a reaction vessel made of an SUS material may be used. To prepare the eutectic mixture, serine and arginine are prepared at a molar ratio of 2:1 in the case of amino acid-amino acid, and serine and malic acid are prepared at a molar ratio of 2:1 in the case of amino acid-AHA. The amount of water may be 25 to 99 parts by weight based on the total weight of the prepared amino acid-amino acid or amino acid-AHA. Preferably, it may be desirable to prepare an amount equal to the total weight of amino acid-amino acid or amino acid-AHA, that is, such that the content of water is 50 parts by weight with respect to the total weight. The speed of the homo-disper is suitably 600 to 4000 rpm, but may be higher or lower. For example, the speed of the homo-disper may be 600 to 3000 rpm, 600 to 2000 rpm, 600 to 1500 rpm, 800 to 1200 rpm, or 900 to 1100 rpm. The heating may be performed under a temperature condition of 45 to 70°C, for example, 45 to 65°C, 45 to 60°C, or 45 to 55°C. The reaction time is preferably at least 15 minutes or longer, and when homogeneity is confirmed, heating may be stopped and cooling may be performed by leaving it alone.

Furthermore, as a method of confirming the homogeneity of the eutectic mixture obtained as described above,

there are i) a method of confirming whether a transparent mixture is formed without a precipitate when the eutectic mixture, which is an over-melting solution dissolved above the solubility of each component, is checked by being irradiated with light in front of a black background, and

ii) a method of confirming the melting point using a differential scanning calorimetry (DSC) device because a single melting point is formed at a temperature lower than the melting point of each component during the formation of the eutectic mixture.

When the AHA and/or the amino acid are/is completely dissolved, the resulting mixture is clear and homogeneous and maintains its liquid state and transparency even after being subjected to a cooling process. The eutectic mixture prepared as described above may be uniform without phase separation even at a very low temperature, for example, a temperature of less than 0°C, thereby maintaining a transparent liquid phase. Further, the eutectic mixture may be maintained without breaking its bond even though the solidification and melting processes are repeated.

The cosmetic composition according to the present invention may include the eutectic mixture in an amount of 0.01 to 50 parts by weight, for example, 0.05 to 40 parts by weight, 0.1 to 30 parts by weight, 0.5 to 25 parts by weight, 0.1 to 50 parts by weight, 1 to 35 parts by weight, 5 to 30 parts by weight, 0.5 to 40 parts by weight, 1 to 30 parts by weight, 5 to 28 parts by weight, 8 to 25 parts by weight, 1 to 50 parts by weight, 5 to 40 parts by weight, 10 to 35 parts by weight, 15 to 25 parts by weight, 18 to 25 parts by weight, 15 to 20 parts by weight, 10 to 20 parts by weight, 10 to 30 parts by weight, 10 to 40 parts by weight, 15 to 35 parts by weight, or 20 to 30 parts by weight, with respect to the total weight of the cosmetic composition, but the amount is not limited thereto. The content of the eutectic mixture may be appropriately adjusted for the formulation of the cosmetic composition.

In the following examples, it was confirmed that even when a high content of the eutectic mixture was contained in the cosmetic, low skin irritation and high skin permeability were exhibited.

As described above, the cosmetic composition including the eutectic mixture according to the present invention may exhibit skin improvement effects such as an excellent keratin exfoliation promoting effect, an effect of reducing the number of pores, an elasticity enhancing effect and a wrinkle alleviation effect, and a skin regeneration promotion effect.

The cosmetic composition according to the present invention may further include an oily component. The oily component is included in the composition to be present in the external phase of the eutectic mixture, thereby helping the eutectic mixture to be stable in the composition.

The oily component may be at least one of oil or wax. All of the oils and waxes typically used as components of cosmetics in the art may be applied. For example, a silicone-based oil, an ester-based oil, a triglyceride-based oil, a hydrocarbon-based oil, or a vegetable oil may be used as the oil, and one or more of these components may be blended, if necessary.

For example, as the silicone-based oil, a silicone-based fluidic oil or a silicone-based crosspolymer dispersed in oil, and the like may be used. For example, as the silicone-based fluid oil, it is possible to use cyclopentasiloxane, cyclohexasiloxane, cycloheptasiloxane, cyclomethicone, cyclophenylmethicone, cyclotetrasiloxane, cyclotrisiloxane, dimethicone, capryldimethicone, caprylyl trimethicone, caprylyl methicone, cetearyl methicone, hexadecyl methicone, hexyl methicone, lauryl methicone, myristyl methicone, phenyl methicone, stearyl methicone, stearyl dimethicone, trifluoropropyl methicone, cetyldimethicone, diphenylsiloxyphenyltrimethicone, dimethylpolysiloxane, methylphenylpolysiloxane, decamethylcyclopentasiloxane, methyltrimethicone or phenyltrimethicone, and the like. The silicone-based oil components may be used either alone or in combination of two or more types of oils. As the silicone-based crosspolymer, a dimethicone crosspolymer, a dimethicone/vinyl dimethicone crosspolymer, a dimethicone PG-10/15 crosspolymer or a PEG-12 dimethicone/PG-20 crosspolymer may be used, but the silicon-based crosspolymer is not limited thereto.

As ester-based oil, it is possible to use ascorbyl palmitate, ascorbyl linoleate, ascorbyl stearate, diisostearyl malate, benzyl benzoate, benzyl laurate, butylene glycol dicaprylate/dicaprate, butylene glycol diisononanoate, butylene glycol laurate, butylene glycol stearate, butyl isostearate, cetearyl isononanoate, cetearyl nonanoate, cetyl caprylate, cetyl ethyl hexanoate, cetylisononanoate, ethylhexyl caprylate/caprate, ethylhexylisononanoate, ethylhexylisostearate, ethylhexyl laurate, hexyl laurate, octyldodecyl isostearate, isopropyl isostearate, isostearyl isononanoate, isostearyl isostearate, isocetylethyl hexanoate, neopentyl glycol dicaprate, neopentyl glycol diethylhexanoate, neopentyl glycol diisononanoate, neopentyl glycol diisostearate, pentaerythrityl stearate, pentaerythrityl tetraethyl hexanoate, dipentaerythrityl hexa acid ester, polyglyceryl-2 diisostearate, polyglyceryl-2 sesquiisostearate, polyglyceryl-2 isostearate, polyglyceryl-2 tetraisostearate, polyglyceryl-2 triisostearate, polyglyceryl-3 diisostearate, polyglyceryl-3 isostearate, polyglyceryl-4 diisostearate, polyglyceryl-4 isostearate, polyglyceryl-6 diisostearate, polyglyceryl-6 sesquiisostearate or triethylhexanoin, and the like.

As the triglyceride-based oil, a C8-C12 acid triglyceride, a C12-C18 acid triglyceride, caprylic/capric/triglyceride, caprylic/capric/lauric triglyceride, a C10-C40 isoalkyl acid triglyceride, a C10-C18 triglyceride, glyceryl triacetyl hydroxystearate, soybean glyceride, tribehenin, tricaprin, triethylhexanoin, triheptanoin, triisostearin, tripalmitin or tristearin may be used.

As the hydrocarbon-based oil, fluid paraffin (liquid paraffin, mineral oil), paraffin, Vaseline, microcrystalline wax or squalene may be used.

As the vegetable oil, avocado oil, wheat germ oil, rosehip oil, shea butter, almond oil, olive oil, macadamia oil, argan oil, meadowfoam oil, sunflower oil, castor oil, camellia oil, corn oil, safflower oil, soybean oil, rapeseed oil, macadamia nut oil, jojoba oil, palm oil, palm kernel oil or coconut oil may be used.

As the wax, any wax such as hydrocarbon-based wax, vegetable wax, or silicone wax generally used in cosmetics may be used. Examples thereof may include candelilla wax, carnauba wax, rice wax, beeswax, lanolin, ozokerite, ceresin wax, paraffin wax, microcrystalline wax, C30-C45 alkyldimethylsilylpolypropylsilsesquioxane, an ethylene/propylene copolymer or polyethylene wax, but are not limited thereto.

The content of the oily component is not particularly limited, and may be included as the remaining content except for the eutectic mixture described above in the composition.

The cosmetic composition of the present invention may further include all kinds of components usable in typical cosmetics, such as a moisturizing agent such as glycerin, butylene glycol, propylene glycol, hexanediol, methyl gluceth-20, diglycerin, and ethylhexylglycerin; a sunscreen such as ethylhexyl methoxycinnamate, ethylhexyl salicylate, ethylhexyl triazone, octocrylene, and bisethylhexyloxyphenol methoxyphenyl triazine; a pH adjusting agent such as triethanolamine; a thickener such as carbomer, xanthan gum, an acrylate/C10-30 alkylacrylate crosspolymer, and hyaluronic acid; a preservative such as phenoxyethanol, methylparaben, and propylparaben; an antioxidant such as BHT, ethyl ascorbyl ether, and ascorbic acid; a skin conditioning agent such as beta-glucan; a surfactant such as cetearyl glucoside and sorbitan stearate; a fragrance or a colorant, and the component is not limited.

Each of the above components included in a cosmetic composition according to the present invention may be included in the cosmetic composition of the present invention, preferably within the range not exceeding the maximum amount stipulated in the "Cosmeceutical Safety and Technical Code" prescribed by the Chinese government.

The cosmetic composition according to the present invention may be prepared in any formulation typically prepared in the art. For example, the cosmetic composition may include a lotion such as a flexible lotion or a nourishing lotion, a spray type lotion, an emulsion such as a facial lotion or a body lotion, a cream such as a nourishing cream, a moisture cream, and an eye cream, a stick, an essence, a cosmetic ointment, spray, gel, a pack, sunscreen, makeup base, a foundation such as liquid or spray type, a powder, a cleansing lotion, a makeup remover such as cleansing oil, a cleansing agent such as cleansing foam, soap, and body wash, but is not limited thereto.

In one exemplary embodiment, the formulation of the cosmetic composition may be a balm, water in oil (W/O), oil in water (O/W), solubilized formulation, or oil formulation.

As used herein, the "solubilized formulation" refers to a formulation in which a small amount of oil component is transparently dissolved in water, and is a formulation showing a transparent phase because the oil drop has a diameter smaller than the wavelength of visible light, and thus the straightness of light is not disturbed by scattering or reflection. The cosmetic composition having the solubilized formulation may be a transparent skin toner, hair tonic, or hair liquid, but the type is not limited thereto.

The cosmetic composition of the present invention may be used according to the typical method of use, and the number of times of use may vary depending on the skin condition or taste of the user.

In addition, the present invention provides a method of preparing the cosmetic composition.

In one exemplary embodiment, the method of preparing the cosmetic composition includes: preparing a eutectic mixture by mixing water, an amino acid and an alpha-hydroxy acid; and

preparing a cosmetic composition including the eutectic mixture.

In the preparation method, water, the amino acid, the alpha-hydroxy acid, the eutectic mixture and the cosmetic composition may be applied as described above.

In addition, the preparing of the eutectic mixture may be performed under homogenization conditions of 600 to 4000 rpm and 45 to 70°C. According to the present invention, the components constituting the eutectic mixture are uniformly mixed under the homogenization conditions as described above, so the skin improvement effects of the eutectic mixture as described above, hypoallergenicity to the skin, and excellent formulation stability can be implemented.

In another exemplary embodiment, the method of preparing the cosmetic composition includes: preparing a eutectic mixture by mixing a first amino acid and a second amino acid; and

preparing a cosmetic composition including the eutectic mixture.

Furthermore, the preparing of the eutectic mixture may include a step of further including water.

In the preparation method, water, the first amino acid, the second amino acid, water, the eutectic mixture and the cosmetic composition may be applied as described above.

Further, the preparing of the eutectic mixture may be performed under homogenization conditions of 600 to 4000 rpm and 45 to 70°C. According to the present invention, the components constituting the eutectic mixture are uniformly mixed under the homogenization conditions as described above, so the skin improvement effects of the eutectic mixture as described above, hypoallergenicity to the skin, and excellent formulation stability can be implemented.

The benefits and features of the present invention, and the methods of achieving the benefits and features will become apparent with reference to experimental examples and preparation examples to be described below in detail. However, the present invention is not limited to the experimental examples and the preparation examples to be disclosed below and may be implemented in various other forms, and the present invention is provided for rendering the disclosure of the present invention complete and for fully informing the scope of the present invention to a person with ordinary skill in the art to which the present invention pertains.

[Advantageous Effects]

The cosmetic composition according to the present invention can exhibit an excellent keratin exfoliation effect, an effect of reducing the number of pores, a skin elasticity enhancing effect, and a skin regeneration promotion effect by including an alpha-hydroxy acid (AHA), an amino acid, and water; or two types of amino acids in the form of a eutectic mixture to improve the skin permeability of the AHA and/or the amino acid without skin irritation at weakly acidic or neutral pH conditions, and can be stably and uniformly maintained without precipitation of an amino acid even at low temperature.

[Description of Drawings]

FIG. 1 is a set of results confirming whether the melting point of the eutectic mixture according to the present invention is below -20°C, and illustrates the results of (A) an aqueous serine solution containing a high content of serine (Comparative Example 1), (B) a mixture of serine and malic acid (Comparative Example 2), (C) the eutectic mixture of the present invention (Example 1), (D) an aqueous serine solution containing a low content of serine (Comparative Example 3), and (E) an aqueous solution of the eutectic mixture of the present invention (Example 2).

FIG. 2 illustrates the results of confirming the degree of skin permeation depending on whether or not the eutectic mixture of the present invention is formed.

FIG. 3A illustrates the change in viscosity depending on the molar ratio of serine and arginine, and FIG. 3B illustrates the change in conductivity depending on the molar ratio of serine and arginine.

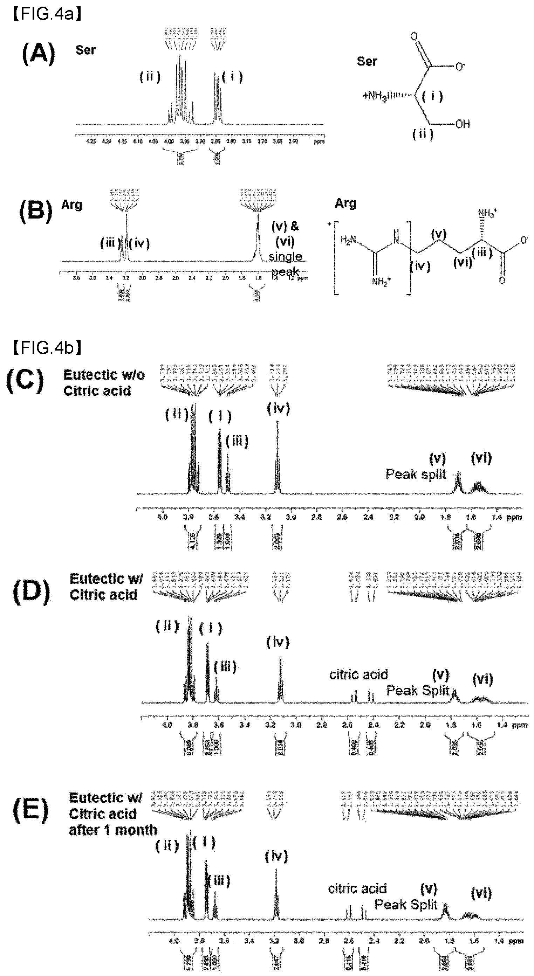

A of FIG. 4A illustrates the NMR analysis results of serine (Ser), and B of FIG. 4A illustrates the NMR analysis results of arginine (Arg).

C of FIG. 4B illustrates the NMR analysis results of a eutectic mixture including serine and arginine, D of FIG. 4B illustrates the NMR analysis results when citric acid is added to a eutectic mixture including serine and arginine, and E illustrates the NMR analysis results after adding citric acid to a eutectic mixture including serine and arginine and storing the resulting mixture under severe conditions (40°C) for 1 month.

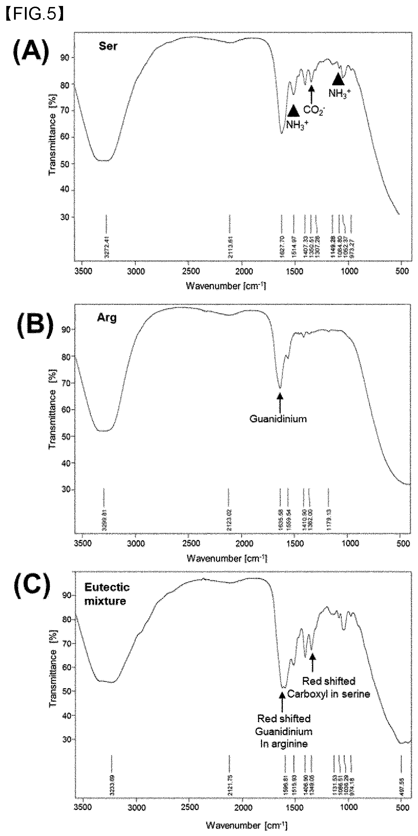

FIG. 5A illustrates the IR spectrum analysis results of serine (Ser), FIG. 5B illustrates the IR spectrum analysis results of arginine (Arg), and FIG. 5C illustrates the IR spectrum analysis results of a eutectic mixture of serine and arginine.

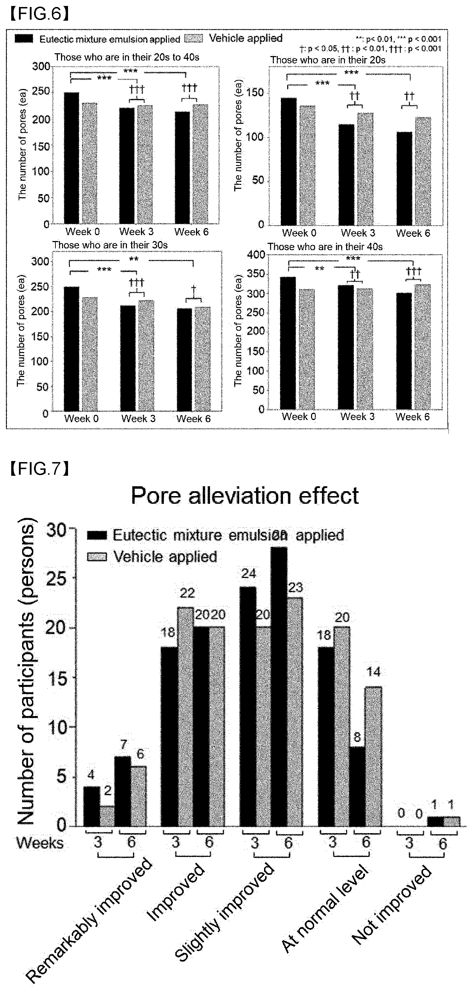

FIG. 6 is a set of results confirming the pore number reducing effect of an essence (emulsion) including a serine-arginine eutectic mixture for participants in the age range 20s to 40s.

FIG. 7 illustrates the results of sensory evaluation (sensory effect evaluation) for the pore number reducing effect of an essence (emulsion) including a serine-arginine eutectic mixture for participants in the age range 20s to 40s.

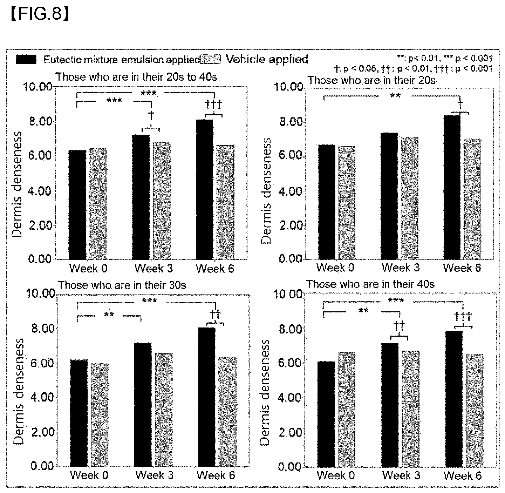

FIG. 8 is a set of results of measuring the dermis denseness of participants in their 20s to 40s according to the treatment with an essence (emulsion) including a serine-arginine eutectic mixture.

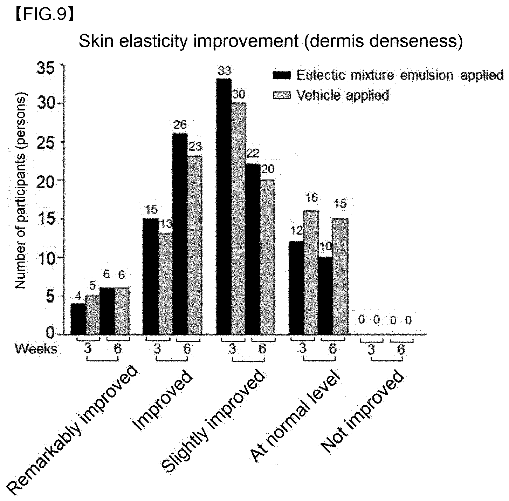

FIG. 9 illustrates the results of sensory evaluation (sensory effect evaluation) for the increase in dermis denseness (increase in skin elasticity) of participants in their 20s to 40s according to the treatment with an essence (emulsion) including a serine-arginine eutectic mixture.

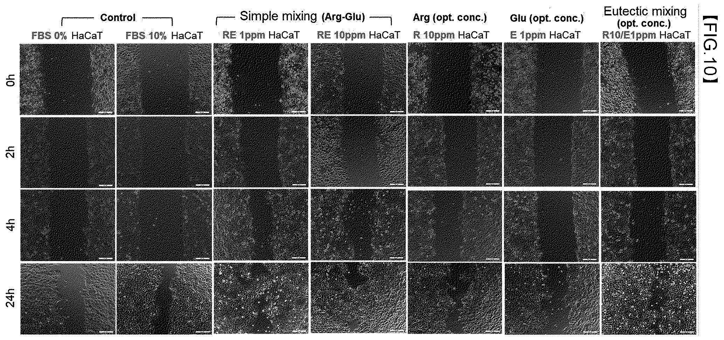

FIG. 10 is a set of results of confirming the skin regeneration promotion effect by treating HaCaT cells, which are a human-derived keratinocyte cell line, with an arginine-glutamic acid eutectic mixture.

[Modes of the Invention]

Hereinafter, the present invention will be described in detail with reference to the following Examples. However, the following Examples are only for exemplifying the present invention, and the content of the present invention is not limited by the following Examples.

**Examples 1 and 2. Preparation of aqueous solution of eutectic mixture including AHA and amino acid**

An aqueous eutectic mixture solution was prepared by the following method.

The eutectic mixture of Example 1 was prepared in a liquid form by preparing 18 parts by weight of malic acid (DL-malic acid, FUSO, Japan) having a melting point of about 130°C based on 100 parts by weight of the eutectic mixture, and heating the malic acid to the melting point. After 20 parts by weight of serine (L-serine, EVONIK, Germany) was added to the liquid malic acid, the resulting mixture was completely melted under stirring at 1,300 rpm for 30 minutes while maintaining the temperature. The completely melted liquid was neutralized with NaOH to have a pH of 6.5. Then, the solution was cooled at room temperature, and a eutectic mixture having a final serine content of 20 parts by weight and a malic acid content of 18 parts by weight was prepared by adding water thereto and stirring the resulting mixture until the content of moisture in the entire eutectic mixture became 53 parts by weight using the Karl Fischer titration method.

The eutectic mixture of Example 2 was prepared by adding 25 parts by weight of the eutectic mixture of Example 1 to water based on 100 parts by weight of the entire eutectic mixture. In this case, serine and malic acid were included in the aqueous solution in an amount of 5 parts by weight and 4.5 parts by weight, respectively.

[Table 1]

| Component (parts by weight) | Example 1 | Example 2 |
|---|---|---|
| Water | 53 | 88.25 |
| Malic acid | 18 | 4.5 |
| Serine | 20 | 5 |
| NaOH | 9 | 2.25 |
| Total | 100 | 100 |

**Comparative Example 1. Preparation of aqueous serine solution including high content of serine**

An aqueous serine solution was prepared by mixing serine (L-Serine, EVONIK, Germany) with water so as to include serine in an amount of 20 parts by weight based on 100 parts by weight of the entire aqueous solution.

**Comparative Example 2. Preparation of aqueous solution of serine and malic acid including high contents of serine and malic acid**

Serine and malic acid were mixed with water and the pH was adjusted to 6.5 using NaOH. A final aqueous solution of the mixture was prepared such that serine (L-Serine, EVONIK, Germany) and malic acid (DL-Malic acid, FUSO, Japan) were included in an amount of 20 parts by weight and 18 parts by weight, respectively, based on 100 parts by weight of the entire mixture.

**Comparative Example 3. Preparation of aqueous serine solution including low content of serine**

An aqueous serine solution was prepared by mixing serine (L-Serine, EVONIK, Germany) with water so as to include serine in an amount of 5 parts by weight based on 100 parts by weight of the entire aqueous solution.

**Comparative Example 4. Preparation of aqueous solution of serine and malic acid including low contents of serine and malic acid**

Serine and malic acid were mixed with water and the pH was adjusted to 6.5 using NaOH. For a final aqueous solution of the mixture, an aqueous solution of serine and malic acid was prepared by mixing serine (L-Serine, EVONIK, Germany) and malic acid (DL-Malic acid, FUSO, Japan) with water so as to include serine and malic acid in an amount of 5 parts by weight and 4.5 parts by weight, respectively, based on 100 parts by weight of the entire mixture.

**Comparative Example 5. Preparation of aqueous malic acid solution**

An aqueous malic acid solution was prepared by mixing malic acid (DL-Malic acid, FUSO, Japan) with water so as to include malic acid in an amount of 4.5 parts by weight based on 100 parts by weight of the entire aqueous solution.

**Examples 3 and 4. Preparation of aqueous solution of eutectic mixture including two types of amino acids**

An aqueous solution of a eutectic mixture including two types of amino acids were prepared by the following method.

A eutectic mixture of Example 3 was prepared by preparing 21 parts by weight of arginine (L-arginine, Daesang Corp., Republic of Korea, melting point approximately 220°C), adding 26 parts by weight of serine (L-serine, EVONIK, Germany) thereto, and then adding 10 parts by weight of water thereto, based on 100 parts by weight of the eutectic mixture, and completely melting the resulting mixture under stirring at 1,300 rpm for 30 minutes while maintaining the temperature at 90°C. A eutectic mixture of Example 4 was prepared by adding 6 parts by weight of citric acid monohydrate (Citric acid, CIBA SPECIALITY CHEMICALS, Switzerland) for adjusting pH to the eutectic mixture of Example 3 and completely melting the resulting mixture under stirring at 1,300 rpm for 30 minutes while maintaining the temperature at 90°C. Then, cooling was performed at room temperature, and then water was added to the eutectic mixtures of Examples 3 and 4 while stirring, wherein the content of moisture in the entire eutectic mixture was matched to 50 to 55 parts by weight using the Karl Fischer titration method. The pH values of Examples 3 and 4 were 8.7 and 6.5, respectively.

[Table 2]

| Component (parts by weight) | Example 3 | Example 4 |
|---|---|---|
| Water | 53 | 52 |
| Arginine | 21 | 15 |
| Serine | 26 | 27 |
| Citric acid monohydrate | 0 | 6 |
| Total | 100 | 100 |

**Experimental Example 1. Confirmation of melting point, low-temperature stability and stability of aqueous eutectic mixture solution**

**1) Confirmation of melting point**

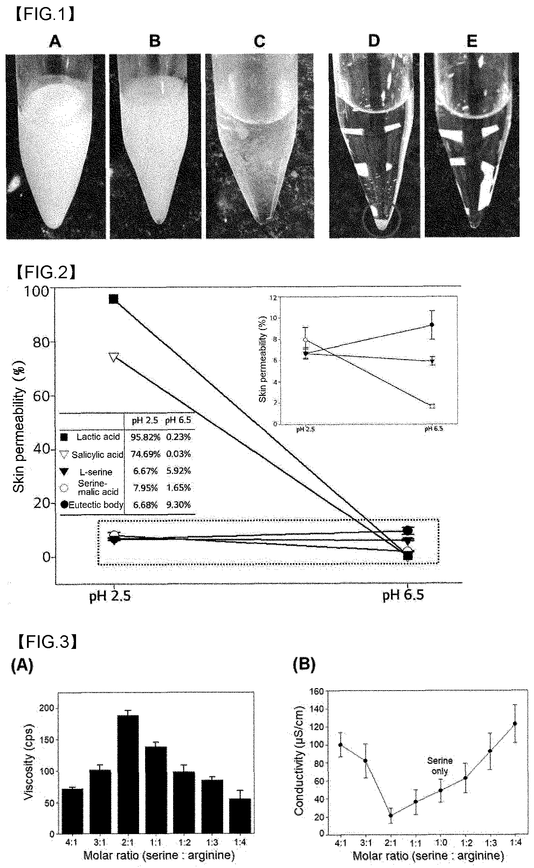

The properties of the aqueous eutectic mixture solution prepared in Example 1, the aqueous serine solution of Comparative Example 1, and the aqueous solution of serine and malic acid of Comparative Example 2 due to a melting point drop were compared. In the experiment, the melting points were compared by allowing the solutions of Example 1 and Comparative Examples 1 and 2 to stand at -20°C to confirm the phase change. The results are illustrated in FIG. 1. In FIG. 1, A, B, and C mean Comparative Example 1, Comparative Example 2, and Example 1, respectively.

As illustrated in FIG. 1, it was confirmed that in the case of the eutectic mixture formed of serine, malic acid and water as in Example 1, the liquid phase at - 20°C was maintained as the melting point became less than -20°C. It was confirmed that the aqueous eutectic mixture solutions prepared in Examples 3 and 4 also had a melting point drop caused by the formation of the eutectic mixture, and thus had a melting point near or below -20°C, which is lower than the melting point of water, 0°C.

## 2) Confirmation of low-temperature stability

To confirm the low-temperature stability of the aqueous eutectic mixture solution, the stability was confirmed with the naked eye by repeating a process of storing the aqueous solutions of Comparative Example 3 and Example 2 frozen at low temperature (-20°C) to completely solidify the aqueous solutions and melting the solidified aqueous solutions at room temperature (25°C) 7 times. The results are illustrated in FIG. 1, and FIGS. 1D and E illustrate the results of the aqueous solution of Comparative Example 3 and the aqueous solution of Example 2, respectively.

As illustrated in FIGS. 1D and E , it was confirmed that in the case of the aqueous serine solution of Comparative Example 3, serine precipitated due to solubility decrease at low temperature, and after melting settled the precipitated serine became a precipitate, whereas in the case of the aqueous solution including the eutectic mixture as in Example 2 precipitation was not seen. Through this, it can be seen that in the aqueous solution including the eutectic mixture, stability can be maintained even at low temperature such as -20°C.

Further, it was confirmed that the aqueous eutectic mixture solutions prepared in Examples 3 and 4 were not precipitated, or re-melted even by slightly stirring at room temperature. Through this, it can be seen that even in Examples 3 and 4 including the eutectic mixture containing two types of amino acids, stability was maintained at a low temperature such as -20°C.

## 3) Confirmation of stability

To confirm the stability of the aqueous eutectic mixture solution, the aqueous serine solution of Comparative Example 1 having the same serine content as that of Example 1 and the aqueous eutectic mixture solutions of Examples 1, 3 and 4 were contained in a 50 ml test tube and allowed to stand for 12 hours for 4 weeks at room temperature (25°C) and -20°C, respectively. The stability of the solution was confirmed by the presence or absence of precipitation in the sample, and the results are shown in the following Table 3.

[Table 3]

| | | | Comparativ e Example 1 | Example 1 | Example 3 | Example 4 |
|---|---|---|---|---|---|---|
| Week 1 | | Room temper ature | Aqueous solution Maintained | Aqueous solution Maintained | Aqueous solution Maintained | Aqueous solution Maintained |
| | | -20°C | Sample precipitation | Aqueous solution Maintained | Aqueous solution Maintained | Aqueous solution Maintained |
| Week 2 | | Room temper ature | Aqueous solution Maintained | Aqueous solution Maintained | Aqueous solution Maintained | Aqueous solution Maintained |
| | | -20°C | Sample precipitation | Aqueous solution Maintained | Aqueous solution Maintained | Aqueous solution Maintained |
| Week 3 | | Room temper ature | Aqueous solution Maintained | Aqueous solution Maintained | Aqueous solution Maintained | Aqueous solution Maintained |
| | | -20°C | Sample precipitation | Aqueous solution Maintained | Aqueous solution Maintained | Aqueous solution Maintained |
| Week 1 | | Room temper ature | Aqueous solution Maintained | Aqueous solution Maintained | Aqueous solution Maintained | Aqueous solution Maintained |
| | | -20°C | Sample precipitation | Aqueous solution Maintained | Aqueous solution Maintained | Aqueous solution Maintained |

As shown in Table 3, even though the aqueous eutectic mixture solutions of Examples 1, 3 and 4 according to the present invention were repeatedly allowed to stand at room temperature and -20°C, precipitation was not observed and the aqueous solution phase was maintained, confirming that stability could be maintained even under various experimental changes. In contrast, Comparative Example 1, which is an aqueous serine solution having the same concentration as in Example 1, showed a result in which the sample was precipitated at -20°C.

**Experimental Example 2. Keratin exfoliation effect of aqueous eutectic mixture solution**

In order to confirm the keratin exfoliation effect of the aqueous eutectic mixture solution by the serine concentration of Example 2, the back skin of a pig was treated with the aqueous solution. Specifically, a 6-mm diameter sample with a thickness of 1 mm was taken from the back skin of the pig using a biopsy tool. After the sample was placed on a 96-well plate and washed once with phosphate buffered saline (PBS), 100 $\mu$l of the aqueous eutectic mixture solution of Example 2 was added thereto. In this case, to compare the keratin exfoliation effects, 100 $\mu$l of Comparative Example 4, a solution containing serine and malic acid, was added to the back skin of a pig in the same manner as described above. After the pig's back skin sample treated with each aqueous solution was stored under conditions of a temperature of 37°C and humidity of 50% for 16 hours, the keratin exfoliation effect was confirmed by measuring the number of exfoliated keratin with a cell counter. In this case, a sample to which water was added was used as a negative control, a sample to which 10% gluconolactone (polyhydroxy acid, PHA) with a pH of 4, which is a known keratin exfoliating substance, was added was used as a first positive control, and a sample to which 10% gluconolactone with a pH of 6 was added was used as a second positive control. In the case of the second positive control, the keratin exfoliation effect was evaluated based on the condition that the efficacy was 15 to 20% compared to the first positive control. Here, the first positive control is applied as a reference for comparing the values between the actual experimental examples, and a relative keratin exfoliation value (%) was calculated and described as [(result value of sample)/(value of first positive control group) $\times$ 100]. The results are shown in Table 4.

[Table 4]

| Application material | pH | Keratin exfoliation Relative value (%) |
|---|---|---|
| **Aqueous solution of [Example 2]: Serine-malic acid eutectic mixture (serine 5 parts by weight, malic acid 4.5 parts by weight)** | **6.5** | **92.6** |
| Composition of [Comparative Example 4]: Simple mixture of serine and malic acid (serine 5 parts by weight, malic acid 4.5 parts by weight) | 6.5 | 82.3 |
| [First positive control] PHA (pH 4.0) | 4.0 | 100 |
| [Second positive control] PHA(pH 6.0) | 6.0 | 18.8 |
| [Negative control] Water | - | 7.1 |

As shown in Table 4, it could be confirmed that a high keratin exfoliation effect was observed in the results of Example 2 in which the aqueous eutectic mixture solution of the present invention was used compared to the aqueous serine solution. In addition, it was confirmed that even though the aqueous solution of Example 2 of the present invention had a higher pH value (pH 6.5) than 10% gluconolactone (PHA) (first positive control) which is a keratin exfoliating component with a pH of 4, the aqueous solution exhibited a similar level of keratin exfoliation effect. That is, the aqueous solution of Example 2 of the present invention can implement a keratin exfoliation effect which is at a similar level to that of the existing exfoliating component having a low pH, or it could be confirmed to implement excellent keratin exfoliation effect even under weakly acidic or neutral pH conditions, which impart less irritation. Furthermore, through the fact that the keratin exfoliation efficacy was increased in the aqueous eutectic mixture solution of the present invention compared to that in the aqueous malic acid solution or the simple serine and malic aicd mixture solution, it was confirmed that a eutectic mixture including water had a better keratin exfoliating effect.

**Experimental Example 3. Confirmation of skin permeability of aqueous eutectic mixture solution under weakly acidic conditions**

The skin permeability of the aqueous eutectic mixture solution was confirmed under weakly acidic conditions (pH 6.5). 10 $\mu$g of each of the aqueous solutions of Comparative Examples 1 and 2 and Example 1 were prepared and applied to pig skin having a uniform area. A tissue soaked with phosphate buffered saline (PBS) was placed on a 6-well plate and stored with the pig skin placed on the tissue under conditions of a temperature of 37°C and a humidity of 50% for 12 hours. Then, the sample that could not penetrate the skin was removed using a cotton swab and a keratin tape, and the weight of the pig skin was measured. After the pig skin was crushed using a homogenizer, the amino acid and the eutectic mixture in the sample were sufficiently dissolved by adding 1 ml of water thereto, and then the resulting solution was centrifuged at 12,000 rpm for 10 minutes. After centrifugation, 0.5 ml of the supernatant was collected, and the serine concentration of the sample, which would be the amount permeated through the skin, was quantified using liquid chromatography with standard samples, and then transmittance was obtained by dividing the quantified concentration by the weight of the pig skin and comparing this to the initially introduced serine mass, as serine amount per gram of the tissue. The results of the skin permeability are illustrated in FIG. 2.

As illustrated in FIG. 2, it could be confirmed that under pH 6.5 conditions, a high pig skin permeability was shown in the results of Example 2 (the eutectic body of FIG. 2) in which the aqueous eutectic mixture solution of the present invention was used compared to the aqueous serine solution (L-serine of FIG. 2). Further, as in FIG. 2 and References, it could be confirmed that Example 2 showed excellent skin permeation efficacy compared to lactic acid (Reference 1) which is a type of AHA and salicylic acid which is a type of β-hydroxy acid (hereinafter, referred to as BHA). In addition, the eutectic mixture of the present invention containing water showed high pig skin permeability compared to a simple serine-and-malic acid mixture solution (serine-malic acid of FIG. 2). That is, it could be seen that from the high skin permeability of the eutectic mixture according to the present invention, an excellent keratin exfoliation effect could be achieved as confirmed in the previous Experimental Example 2.

[References]

1. SE Wolverton, α-Hydroxy acids. In Comprehensive Dermatologic Drug Therapy, 3rd edition, Elsevier, 2012, 570.
2. SE Wolverton, α-Hydroxy acids, In Comprehensive Dermatologic Drug Therapy, 3rd edition, Elsevier, 570 (2012)

**Examples 5 to 7: Preparation of aqueous eutectic mixture solution**

The aqueous eutectic mixture solutions of Examples 5 to 7 were prepared with the compositions and contents as shown in the following Table 5. First, malic acid (DL-Malic acid, FUSO, Japan), which has a melting point at 130°C, was prepared and heated to the melting point to prepare a liquid. Proline (L-Proline, Sigma Aldrich, USA), threonine (L-Threonine, Sigma Aldrich, USA) and cysteine (L-cysteine, Sigma Aldrich, USA) were added to the prepared liquid malic acid, and the resulting mixture was completely dissolved under stirring at 1,300 rpm for 30 minutes while maintaining the temperature. When the solution was completely dissolved, the solution was neutralized to pH 6.5 using NaOH, and cooled at room temperature, and the content of moisture in the entire eutectic mixture was measured using the Karl Fischer method, and it was confirmed whether a eutectic mixture was formed and whether the sample precipitated after storage for 4 weeks. The results are shown in the following Table 6. In this case, Example 5 was an aqueous eutectic mixture solution to which proline was added, Example 6 was an aqueous eutectic mixture solution to which threonine was added, and Example 7 was an aqueous eutectic mixture solution to which cysteine was added.

[Table 5]

| Component (parts by weight) | Example 5 | Example 6 | Example 7 |
|---|---|---|---|
| Water | 56 | 54 | 52 |
| Malic acid | 16 | 16 | 16 |
| Proline | 20 | - | - |
| Threonine | - | 20 | - |
| Cysteine | - | - | 20 |
| NaOH | 8 | 10 | 12 |
| Total | 100 | 100 | 100 |

[Table 6]

| | Example 5 | Example 6 | Example 7 |
|---|---|---|---|
| pH | 6.5 | 6.5 | 6.5 |
| Whether eutectic mixture is formed | Formed | Formed | Formed |
| Content of moisture in aqueous eutectic mixture solution (based on 100 parts by weight of entire aqueous solution) | 56 parts by weight | 54 parts by weight | 52 parts by weight |

(continued)

|  | Example 5 | Example 6 | Example 7 |
|---|---|---|---|
| **Whether sample precipitated after week 4** | Not precipitated | Not precipitated | Not precipitated |

**Preparation Example 1. Preparation of cosmetic composition in balm formulation**

According to the compositions shown in the following Table 7, cosmetic compositions in a balm formulation including the aqueous eutectic mixture solution were prepared. Specifically, a cosmetic composition in a balm formulation was prepared by uniformly dissolving an aqueous phase among the following components, uniformly dissolving an oil phase except for the aqueous phase among the total components at 90°C, and then mixing the aqueous phase with the oil phase, and cooling and solidifying the resulting mixture at room temperature.

[Table 7]

| Component (parts by weight) | Preparation Example 1 |
|---|---|
| 1,2-Hexanediol | 0.5 |
| Cyclopentasiloxane | 30 |
| Dimethicone/vinyl dimethicone crosspolymer | 13 |
| Ceresin | 10 |
| Hexyldecyl ethylhexanoate | 15 |
| Dimethicone/Polysilcone-11 | 10 |
| PEG-10 Dimethicone | 3 |
| Sorbitan sesquioleate | 0.5 |
| Example 1 (aqueous solution of serine-malic acid-water eutectic mixture) | 18 |
| Water | - |
| **Total** | **100** |

**Example 8 and Comparative Examples 6 and 7. Preparation of serine cosmetic composition in oil in water formulation**

According to the compositions shown in the following Table 8, cosmetic compositions with an oil in water (O/W) formulation including the aqueous solution of the eutectic mixture or serine were prepared. Specifically, among the following components, an aqueous phase were uniformly dissolved at room temperature, and an oil phase was uniformly dissolved at 90°C. Then, a cosmetic composition in an oil in water formulation was prepared by mixing the oil phase with the dissolved aqueous phase and cooling the resulting mixture.

[Table 8]

| Component (parts by weight) | Example 8 | Comparative Example 6 | Comparative Example 7 |
|---|---|---|---|
| Water | 47.3 | 67.1 | 72.1 |
| 1,2-Hexanediol | 2 | 2 | 2 |
| Glycerin | 10 | 10 | 10 |
| Butylene glycol | 2 | 2 | 2 |
| Cyclopentasiloxane | 7 | 7.2 | 7.2 |
| Dimethicone | 4.5 | 4.5 | 4.5 |
| Polysorbate 60 | 1.5 | 1.5 | 1.5 |
| Carbomer | 0.35 | 0.35 | 0.35 |
| Tromethamine | 0.35 | 0.35 | 0.35 |

(continued)

| Component (parts by weight) | Example 8 | Comparative Example 6 | Comparative Example 7 |
|---|---|---|---|
| Example 1 (Serine content parts by weight in parenthesis) | 25 (5) | - | - |
| Serine | - | 5 | - |
| Total | 100 | 100 | 100 |

**Experimental Example 4. Confirmation of skin permeation effect**

Skin permeation effects were confirmed by the following method using Example 8 and Comparative Examples 6 and 7, which are the cosmetic compositions in an oil in water formulation.

10 $\mu$g of each of Example 8 and Comparative Examples 6 and 7 was prepared and applied to pig skin having a uniform area. A tissue soaked with phosphate buffered saline (PBS) was placed on a 6-well plate and stored with the pig skin placed on the tissue under conditions of a temperature of 37°C and a humidity of 50% for 12 hours. Then, the sample that could not penetrate the skin was removed using a cotton swab and a keratin tape, and the weight of the pig skin was measured. After the pig skin was crushed using a homogenizer, the amino acid and the eutectic mixture in the sample were sufficiently dissolved by adding 1 ml of water thereto, and then the resulting solution was centrifuged at 12,000 rpm for 10 minutes. After centrifugation, 0.5 ml of the supernatant was collected, and the serine concentration of the sample, which would be the amount permeated through the skin, was quantified using liquid chromatography with standard samples, and then transmittance was obtained by dividing the quantified concentration by the weight of the pig skin and comparing this to the initially introduced serine mass, as serine amount per gram of the tissue. The results are shown in Table 9.

[Table 9]

| | Example 8 | Comparative Example 6 | Comparative Example 7 |
|---|---|---|---|
| Amount of serine permeated through pig skin per unit area [$\mu$g/cm$^2$] | 1.81 $\pm$ 0.054 | 1.02 $\pm$ 0.042 | Not detected |

As can be confirmed in Table 9, in the case of Example 8, the skin permeability was improved by about 77% compared to Comparative Example 6 containing the same amount of serine.

**Preparation Example 2. Preparation of cosmetic composition in water in oil formulation**

According to the compositions shown in the following Table 10, cosmetic compositions in a water in oil (W/O) formulation including the aqueous eutectic mixture solution were prepared. Specifically, among the following components, an aqueous phase was uniformly dissolved at room temperature, and an oil phase was uniformly dissolved at 90°C. Then, a cosmetic composition in a water in oil formulation was prepared by mixing the aqueous phase with the dissolved oil phase and cooling the resulting mixture.

[Table 10]

| Component (parts by weight) | Preparation Example 2 |
|---|---|
| Water | 45.4 |
| Example 1 (aqueous solution of serine-malic acid-water eutectic mixture) | 15 |
| Sodium chloride | 1 |
| 1,2-Hexanediol | 0.7 |
| Glycerin | 17 |
| Dipropylene glycol | 5 |
| Dimethicone | 9.5 |
| Diphenylsiloxyphenyltrimethicone | 1 |
| PEG-10 Dimethicone | 0.2 |
| Dimethicone, dimethicone/PEG-10/15 crosspolymer | 5.2 |

(continued)

| Component (parts by weight) | Preparation Example 2 |
|---|---|
| Total | 100 |

**Example 9 and Comparative Example 8. Preparation of cosmetic composition in oil in water formulation**

According to the compositions shown in the following Table 11, cosmetic compositions with an oil in water (O/W) formulation including the aqueous eutectic mixture solution were prepared. Specifically, among the following components, each of aqueous and oil phases was uniformly dissolved at room temperature, and then, a cosmetic composition in an oil in water formulation was prepared by mixing the oil phase with the dissolved aqueous phase.

[Table 11]

| Component (parts by weight) | Example 9 | Comparative Example 8 |
|---|---|---|
| Water | 58.3 | 73.3 |
| Example 1 (aqueous solution of serine-malic acid-water eutectic mixture) | 15 | - |
| 1,2-Hexanediol | 1 | 1 |
| Glycerin | 10 | 10 |
| Butylene glycol | 2 | 2 |
| Cyclopentasiloxane | 7 | 7 |
| Dimethicone | 4.5 | 4.5 |
| Polysorbate 60 | 1.5 | 1.5 |
| Acrylate/C10-30 alkylarylate crosspolymer | 0.35 | 0.35 |
| Tromethamine | 0.35 | 0.35 |
| Total | 100 | 100 |

**Example 10 and Comparative Example 9. Preparation of cosmetic composition in solubilized formulation**

According to the compositions shown in the following Table 12, cosmetic compositions in a solubilized formulation including the aqueous eutectic mixture solution were prepared. Specifically, among the following components, each of an aqueous phase and a small amount of an oil phase was uniformly dissolved at room temperature, and then, a cosmetic composition in a solubilized formulation was prepared by mixing the small amount of the oil phase with the dissolved aqueous phase.

[Table 12]

| Component (parts by weight) | Example 10 | Comparative Example 9 |
|---|---|---|
| Water | 79.7 | 87.7 |
| Example 3 (aqueous solution of serine-arginine eutectic mixture) | 8 | - |
| Denatured alcohol | 2 | 2 |
| Glycerin | 4 | 4 |
| Butylene glycol | 2 | 2 |
| 1,2-Hexanediol | 2 | 2 |
| PEG-40 hydrogenated castor oil | 0.6 | 0.6 |
| Carbomer | 0.8 | 0.8 |
| Tromethamine | 0.8 | 0.8 |
| Perfume | 0.1 | 0.1 |

(continued)

| Component (parts by weight) | Example 10 | Comparative Example 9 |
|---|---|---|
| Total | 100 | 100 |

**Example 11 and Comparative Example 10. Preparation of cosmetic composition in oil formulation**

According to the compositions shown in the following Table 13, cosmetic compositions in an oil formulation including the aqueous eutectic mixture solution were prepared. Specifically, among the following components, a small amount of an aqueous phase and an oil phase were uniformly dissolved at room temperature, and then, a cosmetic composition in an oil formulation was prepared by mixing the small amount of the dissolved aqueous phase with the oil phase.

[Table 13]

| Component (parts by weight) | Example 11 | Comparative Example 10 |
|---|---|---|
| Example 3 (aqueous solution of serine-arginine eutectic mixture) | 0.5 | - |
| Mineral oil | 8 | 8 |
| Olive oil | 10 | 10 |
| Cetyl ethyl hexanoate | 76.6 | 76.6 |
| PEG-30 sorbitan tetraoleate | 4.9 | 4.9 |
| Water | - | 0.5 |
| Total | 100 | 100 |

**Experimental Example 5. Confirmation of skin improvement effect**

A skin improvement effect (skin tone, skin texture, the number of pores and the number of blackheads) was evaluated by applying the cosmetic compositions of Example 9 and Comparative Example 8 prepared as described above to the skin for 9 weeks. The face of a subject was divided in half by a virtual line passing through the nose, and the cosmetic composition of Example 9 was applied to the right-side skin of the subject's face and the cosmetic composition of Comparative Example 8 was applied to the left-side skin of the subject's face. Then, on weeks 3 and 9, the skin improvement effects on the right side and the left side were compared by allowing the subject to look in a mirror, and a sensory evaluation was performed using a 5-point scale. The results are shown in Table 14. In the following Table 14, in the case of week 0, a score of concerns for each applicable item was shown using the 5-point scale. In order to exclude external factors over time, such as season, individual physical conditions, and mood, the change in satisfaction level for each week was not evaluated, and the efficacy of the eutectic mixture is evaluated by comparing only the left and right conditions at each time point.

[Evaluation criteria for skin improvement effect]

<Week 0>

5 points: The skin conditions for the item are very good and there are no concerns
4 points: The skin conditions for the item are relatively good and there are not many concerns
3 points: The skin conditions for the item are normal and there are some concerns
2 points: There are concerns because the skin conditions for the item are not good
1 point: There are big concerns because the skin conditions for the item are poor

<Weeks 3 and 9>

5 points: Very good skin improvement effect on the right side compared to the left side
4 points: Good skin improvement effect on the right side compared to the left side
3 points: It is difficult to experience the skin improvement effect on the right and left sides

2 points: Good skin improvement effect on the left side compared to the right side
1 point: Very good skin improvement effect on the left side compared to the right side

[Table 14]

| Items experienced by consumer | Week 0 | Week 3 | Week 9 |
|---|---|---|---|
| | (n=25) | (n=25) | (n=5) |
| **Pore conditions are generally improved** | - | 3.0 | 3.8 |
| **The number of blackheads which are noticeable is reduced when the subject looks in the mirror** | 2.84 | 3.1 | 4.0 |
| **The number of pores which are noticeable is reduced when the subject looks in the mirror** | 2.32 | 3.0 | 4.2 |
| **The number of pores such as dark dots is reduced** | 2.92 | 3.1 | 4.0 |
| **When the subject touches the skin with his or her hand, the roughness of the skin is reduced.** | 2.72 | 3.3 | 3.4 |
| **The skin smoothness is improved** | 2.72 | 3.3 | 3.4 |
| **The skin tone uniformity is improved** | 2.88 | 3.0 | 3.2 |

In the case of weeks 3 and 9, it can be interpreted that the larger the score is, by employing 3 points as an average, the more the consumer feels the skin improvement sensory effect on the right side to which the eutectic mixture is applied. Thus, as shown in Table 14, it can be confirmed that when the cosmetic of the present invention is applied for 9 weeks, the skin improvement effect is experienced, and particularly, it can be confirmed that skin concerns such as blackheads, the number of pores, and dark pores, which are difficult to obtain tangible results, are dramatically alleviated compared to consumers' concerns.

**Experimental Example 6. Confirmation of experiencing enhanced effect in wash-off type**

After the cosmetic compositions of Examples 10 and 11 and Comparative Examples 9 and 10 prepared as described above were applied to the skin, a sensory improvement effect by a short-term application in the wash-off type was evaluated by being washed off the skin with lukewarm water. A blind test on changes in sensory effect before the product was applied and after the product was washed off was performed by allowing each subject to use a product randomly selected from Example 10 and Comparative Example 9 on day 1 and to use, on day 2, the other product which had not been selected from Example 10 and Comparative Example 9 on day 1, on each day, respectively, and a sensory evaluation was performed using a 5-point scale. In the evaluation, the inventor, who performed the experiment, knew what kind of product the subject selected, and the subject was subjected to the blind test until the questionnaire survey was completed. The same evaluation was performed for Example 11 or Comparative Example 10 in the same manner as described above. Through the above experiment, it was confirmed whether consumers experienced a short-term efficacy depending on the presence or absence of the eutectic mixture in the solubilized formulation (Example 10 and Comparative Example 9) and the oil formulation (Example 11 and Comparative Example 9), which are two different formulations. The results are shown in Tables 15 and 16, respectively.

[Evaluation criteria for skin improvement effect]

∘: Experienced excellent skin improvement effect
△: Experienced normal skin improvement effect
×: Experienced no skin improvement effect

[Table 15]

| Items experienced by consumer | Example 10 | Comparative Example 9 |
|---|---|---|
| Pore conditions are generally improved | ○ | × |
| The number of blackheads which are noticeable is reduced when the subject looks in the mirror | △ | × |
| The number of pores which are noticeable is reduced when the subject looks in the mirror | △ | × |
| The number of pores such as dark dots is reduced | △ | × |
| When the subject touches the skin with his or her hand, the roughness of the skin is reduced. | ○ | △ |
| The skin smoothness is improved | ○ | × |
| The skin tone uniformity is improved | ○ | △ |

[Table 16]

| Items experienced by consumer | Example 11 | Comparative Example 10 |
|---|---|---|
| Pore conditions are generally improved | △ | × |
| The number of blackheads which are noticeable is reduced when the subject looks in the mirror | △ | × |
| The number of pores which are noticeable is reduced when the subject looks in the mirror | △ | × |
| The number of pores such as dark dots is reduced | △ | × |
| When the subject touches the skin with his or her hand, the roughness of the skin is reduced. | △ | △ |
| The skin smoothness is improved | ○ | × |
| The skin tone uniformity is improved | △ | △ |

As shown in Tables 15 and 16, in the case of the cosmetic composition of the present invention, it could be confirmed by sensory evaluation that the overall experienced skin improvement effect was increased after use.

**Example 12. Preparation of eutectic mixture including serine and arginine**

Serine and arginine were prepared as components forming the eutectic mixture at a molar ratio of 2 : 1 and water in the same amount as the total weight thereof was prepared. That is, the amount of water was set such that the content of water was 50 wt% with respect to the total weight. The homo-disper was heated so as to maintain a speed of 1000 rpm and a temperature of 50°C. When homogeneity was confirmed after reaction for 20 minutes, heating was stopped and cooling was performed naturally.

**Experimental Example 7. Confirmation of eutectic mixture including serine and arginine**

**1) Confirmation of physical properties of eutectic mixture**

The characteristics and long-term stability of the composition according to the molar ratio of serine and arginine were compared with the eutectic mixture of Example 12. Specific molar proportions, characteristics and long-term stability are shown in the following Table 17.

[Table 17]

| Sample | Compositio n (molar ratio) | Characteristics | Stability |
|---|---|---|---|
| Serine : Arginine | 4 : 1 | Yellow fluidic liquid | Not stable; solid precipitation within 3 days |
| | 3 : 1 | Yellow viscous liquid | Not stable; solid precipitation within 21 days |
| | 2: 1 (Eutectic mixture formed) | Yellow viscous liquid | Stable; not precipitated within 2 months |
| | 1 : 1 | Yellow viscous liquid | Not stable; solid precipitation within 21 days |
| | 1 : 2 | Yellow fluidic liquid | Not stable; solid precipitation within 14 days |
| | 1 : 3 | Yellow fluidic liquid | Not stable; solid precipitation within 3 days |
| | 1 : 4 | Yellow fluidic liquid | Not stable; solid precipitation within 1 days |

As shown in Table 17, it could be confirmed that serine and arginine were stable when mixed at a molar ratio of 2 : 1 and were not precipitated even after 2 months of storage. Through this, it can be seen that serine and arginine form a eutectic mixture when included at a molar ratio of 2 : 1.

### 2) Confirmation of viscosity and conductivity of eutectic mixture

The viscosity and conductivity of the compositions according to the molar ratio of serine and arginine in Table 17 were confirmed and are shown in FIG 3.

First, referring to FIG. 3A, where the viscosity was confirmed, it could be confirmed that at the molar ratio of 2 : 1 at which the eutectic mixture was formed, the highest viscosity was shown. From this, it can be seen that the viscosity is increased because in the eutectic mixture, serine and arginine are dissolved at a solubility higher than that of the mixture, and thus are in a hyperdissolved state. Further, referring to the conductivity results (B), it can be confirmed that when serine and arginine have a molar ratio of 2 : 1 due to the stabilization of surface charge (that is, the improvement of lipophilicity), a eutectic mixture is formed, thereby reducing the conductivity.

### 3) NMR Analysis

For NMR analysis of the eutectic mixture, NMR analysis of each of serine and arginine (A and B of FIG. 4A) was performed. In addition, NMR analysis results (C of FIG. 4B) of a eutectic mixture including serine and arginine, NMR analysis results (D of FIG. 4B) of a serine-arginine eutectic mixture to which citric acid was added as a pH adjuster capable of making a cosmetic formulation weakly acidic while having a strong surface charge which affects bonding in the formulation, and NMR analysis results (E ofFIG. 4B) after citric acid was added to the eutectic mixture and the resulting mixture was stored under severe conditions (40°C) for 1 month are shown in FIGS. 4A and 4B.

As a result, it could be confirmed that a single peak observed in the NMR analysis result of arginine (B of FIG. 4A) was split into two peaks while being formed as a serine-arginine eutectic mixture. That is, through intermolecular bonds in the serine-arginine eutectic mixture, the split of hydrogen peaks of the main chain, which is the closest to guanidium of arginine, was observed, which may be interpreted as molecules being bonded because the rotational movement of the main chain is inhibited. Furthermore, as a result of performing an NMR analysis by adding citric acid to the serine-arginine eutectic mixture (D of FIG. 4B), even under a condition where the eutectic mixture may be broken by adding citric acid, the peak of the serine-arginine mixture was identically observed, and the same analysis results were shown even after storage under severe conditions (40°C) for 1 month (E of FIG. 4B). Through this, it was proved that the serine-arginine eutectic mixture of the present invention can be is stable even under severe conditions.

### 4) IR spectrum analysis

The IR spectra of serine and arginine (FIGS. 5A and B) and the serine-arginine eutectic mixture were analyzed (FIG. 5). As a result, a peculiar red shift was confirmed on the IR spectrum due to the formation of a eutectic mixture of serine and arginine. This indicates that the guanidium of arginine and the carboxyl group of serine are red-shifted, and it can be seen that the intramolecular bond is loosened by intermolecular bonding (that is, the bond length is increased), and thus red-shifted. Therefore, through the above structural analysis results, it could be confirmed that the intermolecular bonding of the eutectic mixture was confirmed and was structurally stable.

**Experimental Example 8. Confirmation of skin improvement effect**

### 1) Confirmation of effect of reduction in number of pores

60 subjects in their 20s to 40s were allowed to apply an essence (emulsion) including the eutectic mixture of serine and arginine prepared in Example 12 to their skin, and the pore number reduction effect was confirmed by measuring the number of pores for 6 weeks. In this case, for the effect of reduction in the number of pores, the number of pores was determined as the number of pores recognized by the device, wherein reduction occured when the device fails to recognize single pores as pores due to the reduction of size, color or prominence of the pores. Skin condition was deemed to be adapted and same when the subject stayed in a constant temperature and humidity environment for 20 minutes, and after this the same evaluator measured the number of pores by the Antera 3D® CS skin analyzing camera (Miravex), wherein the same point on the left and right cheeks were used for the measurement. That is, the effect of reducing the number of pores means an effect of improving the expanded pores. The results were divided by each age group, and are shown in FIG. 6 and the following Table 18, respectively.

[Table 18]

| Week | Evaluation Content | All participants (20s to 40s) | | Participants in their 20s | | Participants in their 30s | | Participants in their 40s | |
|---|---|---|---|---|---|---|---|---|---|
| | | Example 12 | Placebo | Example 12 | Placebo | Example 12 | Placebo | Example 12 | Placebo |
| 0 | Number of pores | 254.0 | 232.3 | 144.5 | 135.3 | 249.0 | 227.8 | 342.2 | 310.4 |
| 3 | Number of pores | 223.8 | 227.9 | 114.5 | 127.2 | 211.4 | 221.7 | 320.8 | 311.4 |
| | Reduction rate (%) | 15.177*** | 2.060 | 21.764*** | 4.491 | 17.769*** | 2.956 | 6.501** | -1.174 |
| 6 | Number of pores | 213.9 | 226.9 | 106.0 | 122.1 | 205.9 | 208.4 | 300.7 | 322.4 |
| | Reduction rate (%) | 19.317*** | 2.930 | 25.924*** | 6.633 | 21.376** | 8.039 | 11.301*** | - 4.836 |
| **: $p < 0.01$, ***: $p < 0.001$ | | | | | | | | | |

As shown in Table 18 and FIG. 6, it can be seen that the effect of reduction in the number of pores was continuously exhibited in the skin treated with an essence (emulsion) including the serine-arginine eutectic mixture in the experimental participants of all ages.

### 2) Sensory evaluation for reduction in number of pores

In addition, a sensory evaluation was performed for the reduction in the number of pores according to the use of the essence including the serine and arginine eutectic mixture of Example 12. The results are shown in the following FIG. 7.
Similar to the results of 1), it could be confirmed that more participants showed an excellent effect of reduction in the number of pores in a group treated with an essence including a eutectic mixture of serine and arginine.

### 3) Confirmation of elasticity improvement effect - Confirmation of dermis denseness

The effect of improving the dermis denseness by each age group of participants in their 20s and 40s was confirmed in the same manner as in 1), and is illustrated in FIG. 8.
As can be illustrated in FIG. 8, it could be confirmed that the dermis denseness was high in the experimental group treated with the essence including the serine and arginine eutectic mixture in all age groups. That is, through this, it can be seen that according to the above serine-arginine eutectic mixture treatment, an elasticity improvement effect due to the increase in dermis denseness may be exhibited.

## 4) Sensory evaluation for elasticity improvement

Furthermore, a sensory evaluation was performed for the increase in dermis denseness due to the use of the essence including the serine and arginine eutectic mixture of Example 12. The results are illustrated in FIG. 9.
Similar to the results of 3), it could be confirmed that more participants showed an excellent dermis denseness increase effect in an experimental group treated with an essence including a eutectic mixture of serine and arginine.

## Comparative Example 11. Preparation of simple mixture of serine and arginine

5 parts by weight of serine and 4.1 parts by weight of arginine were added to 90.5 parts by weight of water with respect to 100 parts by weight of a mixture, and the mixture was simply mixed using a homo-disper until the precipitate disappeared. Simple mixing means mixing at room temperature without any heating process.

## Experimental Example 9. Keratin exfoliation effect of eutectic mixture

In order to confirm the keratin exfoliation effect of the serine-arginine eutectic mixture of Example 12 and the simple mixture of serine and arginine of Comparative Example 11, the back skin of the pig was treated with the eutectic mixture and the simple mixture. A specific experimental method was performed in the same manner as in Experimental Example 2. The results are shown in the following Table 19.

[Table 19]

| Application material | pH | Keratin exfoliation relative value (%) |
|---|---|---|
| [Example 12]: Serine-arginine eutectic mixture (serine 5 parts by weight, arginine 4.1 parts by weight) | 9.1 | 96.4 |
| Sample in which pH of eutectic mixture of [Example 12] was adjusted to weakly acidic using citric acid (serine 5 parts by weight, arginine 4.1 parts by weight) | 6.0 | 96.2 |
| [Comparative Example 11]: Serine-arginine simple mixture (serine 5 parts by weight, arginine 4.1 parts by weight) | 9.1 | 78.2 |
| Sample in which pH of simple mixture of [Comparative Example 11] was adjusted to weakly acidic using citric acid (serine 5 parts by weight, arginine 4.1 parts by weight) | 6.0 | 81.5 |
| [First positive control] PHA(pH 4.0) | 4.0 | 100 |
| [Second positive control] PHA(pH 6.0) | 6.0 | 25.5 |
| [Negative control] Water | - | 2.1 |

As shown in Table 19, it could be confirmed that a higher keratin exfoliation effect was exhibited in the serine-arginine eutectic mixture of the present invention compared to the simple mixture of serine and arginine. It was confirmed that the skin exfoliation effect of the eutectic mixture according to the present invention was similar to that of PHA despite having a higher pH value (pH 9.1) compared to the first positive control, as in Experimental Example 2. Furthermore, it was confirmed that the experimental group in which the pH was adjusted to 6.0 with citric acid also showed a level of keratin exfoliation effect similar to that of the eutectic mixture before adjustment.

## Experimental Example 10: Confirmation of skin permeability

The skin permeability of the eutectic mixture prepared in Experimental Example 12 was confirmed. A specific experimental method was performed in the same manner as in Experimental Example 3. The results for Example 12 are shown in Table 20.

[Table 20]

| Application material | pH | Skin permeability relative value (Maximum value converted to 100%) |
|---|---|---|
| **[Example 12]: Serine-arginine eutectic mixture (serine 5 parts by weight, arginine 4.1 parts by weight)** | 9.1 | 96.4 |
| **Sample in which pH of eutectic mixture of [Example 12] was adjusted to weakly acidic using citric acid (serine 5 parts by weight, arginine 4.1 parts by weight)** | 6.0 | 100 |
| [Comparative Example 11]: Serine-arginine simple mixture (5 parts by weight of serine, 4.1 parts by weight of arginine) | 9.1 | 46.2 |
| Sample in which pH of simple mixture of [Comparative Example 11] was adjusted to weakly acidic using citric acid (5 parts by weight of serine, 4.1 parts by weight of arginine) | 6.0 | 82.4 |
| Serine (5 parts by weight) | 6.0 | 71.7 |

It was confirmed that, if the case where the pH was adjusted to 6.0 by adding citric acid to the eutectic mixture according to the present invention was converted to 100%, the eutectic mixture of Example 12 according to the present invention was excellent in skin permeability even under a condition of pH 9.1. In contrast, the simple mixture of serine and arginine showed a significantly lower skin permeability than Example 12, and showed a low result compared to the eutectic mixture according to the present invention even when the pH was adjusted to 6.0.

**Example 13. Preparation of eutectic mixture including arginine and glutamic acid**

Serine and glutamic acid were prepared as components forming the eutectic mixture at a molar ratio of 1 : 1 and water in the same amount as the total weight thereof was prepared. That is, the amount of water was set such that the content of water was 50 wt% with respect to the total weight. The homo-disper was heated so as to maintain a speed of 1000 rpm and a temperature of 50°C. When homogeneity was confirmed after reaction for 20 minutes, a eutectic mixture was prepared in the same manner as in Example 12 by stop heating and cooling at room temperature.

**Experimental Example 11. Confirmation of characteristics of eutectic mixture including arginine and glutamic acid**

A macroscopic evaluation of the formation of a eutectic mixture of arginine and glutamic acid was performed. The solubilities of glutamic acid, a simple mixture of arginine and glutamic acid and an arginine-glutamic acid eutectic mixture in water were confirmed, and whether or not 1% or more thereof can be dissolved is shown in the following Table 21.

[Table 21]

| Solute dissolved in water (solvent) | Whether it can be dissolved at 1% or more |
|---|---|
| Glutamic acid | X (solubility < 0.5%) |
| Arginine and glutamic acid simple mixture | X (solubility < 0.5%) |
| Arginine-glutamic acid eutectic mixture | o (solubility about 15.2%) |

As a result, as shown in Table 21, in the case of the glutamic acid and the arginine and glutamic acid simple mixture, the solubilities in water were less than 0.5% which is so low that it is difficult to dissolve 0.5% or more of the glutamic acid and the arginine and glutamic acid simple mixture in an actual formulation such as oil or an aqueous phase. In contrast, in the case of the arginine-glutamic acid eutectic mixture, it was confirmed that the solubility in water was 15.2%, and the eutectic mixture could be present as an aqueous solution phase and could be additionally dissolved by eutectic bonding.

**Experimental Example 12. Confirmation of skin regeneration promotion effect**

HaCaT cells, which are a human-derived keratinocyte cell line, were cultured under conditions of 37°C and 5% $CO_2$ using a DMEM medium (ADDEXBIO TECHNOLOGIES, SanDiego, CA, USA) supplemented with 10% fetal bovine serum (Gibco, Waltham, MA, USA), 100 mg/ml penicillin and 100 mg/ml streptomycin. After the cultured cells were seeded into a 24-well plate so as to have $2.0 \times 10^5$ cells per well, and then cultured for 24 hours, the cells were washed once with PBS, and scratches having a perpendicular line shape were created on the center of the plate by scraping the cells with a 200 $\mu$l pipet tip. The cells were washed twice with PBS to remove the dropped cell debris, and the medium was replaced with a PBS-free DMEM containing each test material. In order to always capture the same part when the cells were photographed, the center of the well was marked with a fine marker and the cell photograph of the marked part was taken at the same time at the start of the experiment (0 hours). The cell photographs after 2, 4 and 24 hours were also taken in the same way. After a scratch area created on monolayer cells was measured from the photographs after 0 hours and 24 hours using an image analysis program (Image J), the area filled with the scratch area was shown as a wound healing percentage (%) according to the following General Formula 1. The results are shown in the following Table 22 and FIG. 10. In this case, the concentration of the Arg and Glu (10 : 1) simple mixture is based on the concentration of arginine.

[General Formula 1]

$$\text{Wound healing percentage (\%)} = \left\{ \frac{\left( \begin{array}{c} \text{Distance between} \\ \text{scratches at 0 hour} \end{array} - \begin{array}{c} \text{Distance between} \\ \text{scratches at 24 hours} \end{array} \right)}{\text{Distance between scratches at 0 hour}} \right\} \times 100$$

[Table 22]

|  | Wound healing percentage (%) after 24 hours |
|---|---|
| FBS 0% (negative control) | 48.6 |
| FBS 10% (positive control) | 72.7 |
| Arg-Glu eutectic mixture 1 ppm* | 88.9 |
| Arg-Glu eutectic mixture 10 ppm | 92.2 |
| Arg 10ppm | 73.5 |
| Glu 1ppm | 70.1 |
| Arg and Glu (10:1) simple mixture 10 ppm* | 80.6 |

As in Table 22 and FIG. 10, as a result of comparing the arginine-glutamic acid mixture (Arg-Glu eutectic mixture), each of single amino acids arginine and glutamic acid, and a simple mixture of two amino acids by employing an FBS-free DMEM medium as a negative control and a 10% FBS-DMEM positive control corresponding to the optimal growth conditions of the cell line as a positive control, it could be confirmed that an Arg-Glu eutectic mixture 1 ppm treatment group showed an excellent wound healing percentage compared to that of an FBS 10% treatment group (positive control). Further, the wound healing percentage of the 10 ppm Arg-Glu eutectic mixture (based on the concentration of arginine) was 92.2%, and it could also be confirmed on the microscopic image that most of the space vacated by scratches was covered with cells. Such an effect of the 10 ppm Arg-Glu eutectic mixture is excellent compared to a group treated with a simple mixture of both amino acids, 10 ppm arginine and 1 ppm glutamic acid (maximum concentration of glutamic acid that can be prepared is 1 ppm) and a group treated with 1 ppm arginine, and through this, it was confirmed that the Arg-Glu eutectic mixture could help skin regeneration by promoting cell regeneration compared to the case where each amino acid is applied alone or where each amino acid is simply mixed.

**Experimental Example 13. Confirmation of effect of increasing total amount of collagen**

In order to confirm the effect of the eutectic mixture of Example 13 on an increase in the total amount of collagen, the total amount of collagen produced from human fibroblasts was measured after the human fibroblasts were treated with the arginine-glutamic acid eutectic mixture. Specifically, a degree of increase in the total amount of collagen at the cell level was confirmed by adding an arginine-glutamic acid eutectic mixture (Arg-Glu eutectic mixture), arginine (Arg), glutamic acid (Glu) and an arginine and glutamic acid simple mixture (Arg/Glu simple mixture) to a culture solution of human fibroblasts. The total amount of collagen was quantified using a PICP kit (Procollagen Type I C-Peptide Enzyme ImmunoAssay KIT). For human-derived fibroblasts before the experiment, the Arg-Glu eutectic mixture was evaluated for cytotoxicity at different concentrations, and a degree of increase in the total amount of collagen was evaluated by selecting a non-cytotoxic concentration (100 $\mu$g/ml).

EP 4 623 891 A2

Specifically, after each sample was added to the culture medium of human fibroblasts and cultured for 1 day, the culture medium was taken, and a degree of increase in the total amount of collagen at each concentration was measured at 450 nm using a spectrophotometer with the PICP EIA kit. For comparison of effects, the degrees of increase in the total amount of collagen for the culture medium of fibroblasts to which nothing was added (negative control) and the sample to which TGF-βfmf was added so as to have a final concentration of 10 mg/ml (positive control) were confirmed in the same manner as described above. The total amount of collagen was measured by UV absorbance, a rate of increase in the total amount of collagen was calculated by a ratio of the total amount of collagen relative to the control, and the results are shown in the following Table 23. In this case, the concentrations of the Arg-Glu eutectic mixture and the Arg and Glu (1 : 10) simple mixture were based on the concentration of arginine.

[Table 23]

| Sample | Average absorbance | Collagen increase rate (%) |
|---|---|---|
| Control (no additive) | 2.036 | - |
| TGF-β 10 ng/ml | 2.335 | 14.7 |
| Arg-Glu eutectic mixture 10 ppm | 2.728 | 34.0 |
| Arg 10 ppm | 2.429 | 19.3 |
| Glu 10 ppm | 2.240 | 10.0 |
| Arg/Glu (1:10) simple mixture 10 ppm | 2.531 | 24.3 |

Effect of increasing total amount of collagen (number of repeats = 3)

As can be seen in Table 23, the Arg-Glu eutectic mixture showed an effect of increasing the total amount of collagen by promoting collagen synthesis in a concentration-dependent manner, and showed an excellent rate of increase in collagen at a concentration of 1 ppm or more compared to the Arg and Glu (1:10) simple mixture at the same concentration. Furthermore, it could be confirmed that the Arg-Glu eutectic mixture also exhibited an excellent rate of increase in collagen compared to simple amino acids Arg and Glu.

**Experimental Example 14. Confirmation of skin elasticity improvement effect**

In order to confirm the elasticity enhancing effect of the eutectic mixture of Example 13 on real human skin, an experiment was performed by preparing A and B in a cream formulation according to the compositions of the following Table 24. In the present experiment, an arginine-glutamic acid eutectic mixture (Arg-Glu eutectic mixture) was used. Specifically, 20 women aged 25 to 45 years were allowed to apply the cream formulations to the face twice daily in the morning and evening, and a skin elasticity improvement effect was measured using a skin elasticity measuring device (Cutometer SEM 575, C+K Electronic Co., Germany). The results are shown in the following Table 25. The result value refers to the skin viscoelasticity of the skin elasticity measuring device.

[Table 24]

| Component | A | B |
|---|---|---|
| Arg-Glu eutectic mixture | 1 | 0 |
| Water | Up to 100 | Up to 100 |
| Glycerin | 8.0 | 8.0 |
| Butylene glycol | 4.0 | 4.0 |
| Hyaluronic acid extract | 5.0 | 5.0 |
| Beta-glucan | 7.0 | 7.0 |
| Carbomer | 0.15 | 0.15 |
| Caprylic/Capric triglyceride | 8.0 | 8.0 |
| Squalene | 5.0 | 5.0 |
| Cetearyl glucoside | 1.5 | 1.5 |

(continued)

| Component | A | B |
|---|---|---|
| Sorbitan stearate | 0.4 | 0.4 |
| Cetearyl alcohol | 2.0 | 2.0 |
| Preservative | Appropriate amount | Appropriate amount |
| Colorant | Appropriate amount | Appropriate amount |
| Triethanolamine | 0.15 | 0.15 |
| Total | 100 | 100 |

[Table 25]

| | Rate (%) of change in skin elasticity after week 8 |
|---|---|
| A | 29 |
| B | 12 |

As shown in Table 25, it could be confirmed that the skin elasticity was increased in an experimental group treated with the arginine-glutamic acid eutectic mixture. Through this, it can be seen that the arginine-glutamic acid eutectic mixture showed an excellent skin elasticity enhancing effect.

**Experimental Example 15. Confirmation of separation conditions for eutectic mixture**

In general, when a eutectic mixture is formed in a specific solvent, the eutectic mixture is placed in a state of being overmelted at a level equal to or more than the solubility of each component species. In the present invention, it can be confirmed that in the case of an overmelted solution that forms a eutectic mixture using water as a solvent, the bond of the eutectic mixture is dissociated depending on whether an irreversible precipitate is generated, for example, under the following conditions:

i) When the eutectic mixture is stored under a temperature condition of 50°C for 6 weeks or more, 8 weeks or more, 10 weeks or more, or 12 weeks or more;
ii) When the eutectic mixture is stored under a temperature condition of 60°C for 4 weeks or more, 5 weeks or more, 6 weeks or more, or 8 weeks or more; and/or
iii) When the eutectic mixture is stored under a temperature condition of 80°C for 10 days or more, 15 days or more, 20 days or more, or 30 days or more.

That is, under the conditions of i) to iii), it is possible to break the bond between the eutectic materials in the eutectic mixture through the presence or absence of the generation of an irreversible precipitate, and it can be confirmed that the bond is broken. In addition, whether the eutectic mixture is dissociated can be confirmed using a material capable of breaking the eutectic materials, for example, a material such as EDTA, a protic solvent, a high concentration of urea, or guanidyl HCl.

In this case, reversible precipitation means precipitation that returns to a clear (homogeneous) solution state by simply mixing (handshaking) a eutectic mixture about 10 to 20 times after the precipitate of the eutectic mixture is observed, and it was confirmed that precipitation occurred temporarily when the mixture was allowed to stand at a low temperature (-20°C) instead of the dissociation conditions of i) to iii), but it was confirmed that homogenization was achieved by simple mixing. Irreversible precipitation means precipitation that is not homogenized when it is subjected to the above process.

Furthermore, it was confirmed whether the eutectic mixture was dissociated by observing the properties of a cosmetic (essence) including the eutectic mixture of Example 12, which was allowed to stand under separation conditions of the eutectic composition (Table 27) and confirming changes in melting point through measurement by differential scanning calorimetry (DSC) (Table 28) and the keratin exfoliation effect (Table 29), of the cosmetic. In this case, the essence including the eutectic mixture means an essence obtained by adding the eutectic mixture to an essence prepared by a composition shown in the following Table 26, and the keratin exfoliation effect was performed in the same manner as that described in Experimental Example 2. For the measurement of the melting point, Perkin Elmer Diamond DSC (Differential Scanning Calorimeter; Perkin Elmer, Waltham, MA, USA) was used. The melting point is expressed as a heat flow with respect to the sample in the DSC, and its accuracy is known to be 0.01°C. The melting point was measured in a pure nitrogen atmosphere, nitrogen was fed into a measuring chamber at a rate of 20 cc/min in order to maintain the nitrogen atmosphere, and the temperature of the chamber was set so as to increase by 10°C per minute. The mass of the measured sample was confirmed by subtracting the weight of a pan for accommodating the sample in the DSC from the weight including the entire sample. The heat flow was measured by placing the pan inside the DSC, setting the temperature from -50°C to 90°C, and heating the pan. Subsequent results were confirmed with a data analysis program connected to the DSC.

[Table 26]

| Component | Content (parts by weight) |
|---|---|
| Isohexadecane | 5.0 |
| Glycerin | 3.6 |
| Water | 83.94 |
| 1,2-Hexanediol | 1.0 |
| Propanediol | 6.0 |
| Xanthan gum | 0.1 |
| Acrylate/C10-30 alkylarylate crosspolymer | 0.2 |
| Perfume | 0.06 |
| Tromethamine | 0.1 |
| Total | 100 |

[Table 27]

| Separation conditions | Properties |
|---|---|
| At 50°C for 6 weeks | Occurrence of irreversible precipitation (separation of eutectic mixture) |
| At 60°C for 5 weeks | Occurrence of irreversible precipitation (separation of eutectic mixture) |

As shown in Table 27, it could be confirmed through the fact that irreversible precipitation occurred when the cosmetic was allowed to stand under a temperature condition of 50°C for 6 weeks or more or under a temperature condition of 60°C for 5 weeks or more that the eutectic mixture was dissociated. Since the effects of lowering the melting point and improving skin permeation efficacy cannot be expected when the eutectic mixture is dissociated, the characteristics when the eutectic mixture was formed or dissociated were confirmed through the following Tables 28 and 29.

[Table 28]

| Application sample in essence | Melting point of aqueous phase |
|---|---|
| Essence to which no eutectic mixture is added (control) | 0°C |
| **Application of eutectic mixture of [Example 12] (serine-arginine, serine 5 parts by weight, arginine 4.1 parts by weight)** | - 3.83°C |
| Application of simple mixture of [Comparative Example 11] (serine-arginine, serine 5 parts by weight, arginine 4.1 parts by weight) | 0°C |
| **Application of eutectic mixture of [Example 12] (serine-arginine, serine 5 parts by weight, arginine 4.1 parts), and then storage at 50°C for 6 weeks** | 0°C |

As a result, the melting point of the simple mixture, which does not form the eutectic mixture, was confirmed at 0°C, which is the melting point of water, but the melting point of the cosmetic including the eutectic mixture was confirmed at - 3.83°C due to a melting point dropping phenomenon. In the case of the cosmetic including the eutectic mixture, which was stored under the dissociation conditions (at 50°C for 6 weeks) of Table 27, it could be confirmed that the eutectic mixture was dissociated, and thus became a simple mixture because the melting point was observed at 0°C.

[Table 29]

| Application sample in essence | Keratin exfoliation efficacy |
|---|---|
| Essence to which no eutectic mixture is added (control) | 12.5 |
| **Application of eutectic mixture of [Example 12] (serine-arginine, serine 5 parts by weight, arginine 4.1 parts by weight)** | 100.0 |
| Application of simple mixture of [Comparative Example 11] (serine-arginine, serine 5 parts by weight, arginine 4.1 parts by weight) | 82.5 |
| **Application of eutectic mixture of [Example 12] (serine-arginine, serine 5 parts by weight, arginine 4.1 parts by weight) and then storage at 50°C for 6 weeks** | 86.2 |

When the eutectic mixture confirmed in Table 28 was dissociated and thus became a simple mixture, it was confirmed by Table 29 whether the level of the keratin exfoliation effect was similar to that of the simple mixture. When a relative comparison was made by setting the keratin exfoliation effect of the essence including the eutectic mixture to 100, it was confirmed that all the essences including a dissociated product of the simple mixture or the eutectic mixture had similar levels. In this case, the essence including the eutectic mixture of [Example 12] exhibited a keratin exfoliation effect that was at least approximately 20% or more higher than an essence (including the dissociated product of the eutectic mixture) obtained after the essence including the eutectic mixture of [Example 12] was stored at 50°C for 6 weeks (severe conditions).

As can be seen in Tables 28 and 29, it could be confirmed that when the melting points and keratin exfoliation effects of the essence including the eutectic mixture and the essence including the dissociated product of the eutectic mixture were compared, the dissociated product of the eutectic mixture exhibited a melting point and keratin exfoliation effect similar to those of the essence including the simple mixture. That is, through the results, it was proved that it could be confirmed whether the eutectic mixture was dissociated.

## Claims

1. A cosmetic composition comprising a eutectic mixture containing a first amino acid; and a second amino acid, wherein the first amino acid and the second amino acid are arginine and glutamic acid.

2. The cosmetic composition of claim 1, wherein the first amino acid and the second amino acid are comprised in a total amount of 1 part by weight to 75 parts by weight with respect to the total 100 parts by weight of the eutectic mixture.

3. The cosmetic composition of claim 1, wherein the eutectic mixture further comprises water.

4. The cosmetic composition of claim 3, wherein water is comprised in an amount of 25 parts by weight to 80 parts by weight with respect to the total 100 parts by weight of the eutectic mixture.

5. The cosmetic composition of claim 1, wherein the eutectic mixture is comprised in an amount of 0.01 part by weight to 50 parts by weight with respect to the total 100 parts by weight of the entire composition.

6. The cosmetic composition of claim 1, wherein the cosmetic composition has a pH value of 3.5 to 10.

7. The cosmetic composition of claim 1, wherein the cosmetic composition is for alleviating pores by reducing the number of pores.

8. The cosmetic composition of claim 1, wherein the cosmetic composition is for enhancing skin elasticity.

9. The cosmetic composition of claim 1, wherein the cosmetic composition is for regenerating the skin.

10. The cosmetic composition of claim 1, wherein the cosmetic composition is for alleviating wrinkles.

11. The cosmetic composition of claim 1, wherein the cosmetic composition is for improving keratin exfoliation.

12. A method of preparing the cosmetic composition of claim 1, the method comprising:

preparing a eutectic mixture by mixing the first amino acid and the second amino acid; and
preparing the cosmetic composition by adding the eutectic mixture.

13. The method of claim 12, wherein the preparing of the eutectic mixture is performed under homogenization conditions of 600 to 4000 rpm and 45 to 70°C.

14. The method of claim 12, wherein the preparing of the eutectic mixture comprises a step of further comprising water.

【FIG.1】

A    B    C    D    E

【FIG.2】

|  | pH 2.5 | pH 6.5 |
|---|---|---|
| ■ Lactic acid | 95.82% | 0.23% |
| ▽ Salicylic acid | 74.69% | 0.03% |
| ▼ L-serine | 6.67% | 5.92% |
| ○ Serine-malic acid | 7.95% | 1.65% |
| ● Eutectic body | 6.68% | 9.30% |

【FIG.3】

(A)

(B)

【FIG.4a】

【FIG.4b】

【FIG.5】

【FIG.6】

【FIG.7】

## Pore alleviation effect

【FIG.8】

【FIG.9】

Skin elasticity improvement (dermis denseness)

【FIG.10】

REFERENCES CITED IN THE DESCRIPTION

**Patent documents cited in the description**

- KR 101508168

**Non-patent literature cited in the description**

- *Journal of the Korean Society for Wellness*, 2015, vol. 10 (2), 161-169
- **TATSUYA OZAWA et al.** The role of humectants in skin moisture retention. *Skin Research*, 1985, vol. 27, 276-288
- S. E. coli Wolverton, $\alpha$-hydroxy acids. Comprehensive Dermatologic Drug Therapy. Elsevier, 2012, vol. 570
- $\alpha$-Hydroxy acids. **SE WOLVERTON**. Comprehensive Dermatologic Drug Therapy,. Elsevier, 2012, vol. 570
- $\alpha$-Hydroxy acids. **SE WOLVERTON**. Comprehensive Dermatologic Drug Therapy. Elsevier, 2012, vol. 570